Europäisches Patentamt

European Patent Office

Office européen des brevets

(11) Publication number: **0 044 710**
**B2**

(12) NEW EUROPEAN PATENT SPECIFICATION

(45) Date of publication of the new patent specification: **06.02.91**

(21) Application number: **81303266.1**

(22) Date of filing: **16.07.81**

(51) Int. Cl.⁵: **C 07 K 7/08, A 61 K 39/395, A 61 K 39/02, A 61 K 37/02**

(54) **Synthetic peptide specific antigenic determinant and method of manufacturing antigenic materials therefrom.**

(30) Priority: **17.07.80 US 169758**
**30.10.80 US 202431**
**27.03.81 US 248059**

(43) Date of publication of application:
**27.01.82 Bulletin 82/04**

(45) Publication of the grant of the patent:
**06.02.91 Bulletin 91/06**

(45) Mention of the opposition decision:
**02.04.86 Bulletin 86/14**

(84) Designated Contracting States:
**AT BE CH DE FR GB IT LI NL SE**

(56) References cited:
**EP-A-0 001 930**
**EP-A-0 013 828**
**EP-A-0 015 059**
**EP-A-0 020 251**
**DE-A-3 014 582**
**US-A-4 075 194**
**US-A-4 193 915**
**US-A-4 237 224**

The file contains technical information submitted after the application was filed and not included in this specification

(73) Proprietor: **SCRIPPS CLINIC AND RESEARCH FOUNDATION**
**10666 North Torrey Pines Road**
**La Jolla California 92037 (US)**

(72) Inventor: **Lerner, Richard Alan**
**7750 E. Roseland**
**La Jolla California 92037 (US)**
Inventor: **Green, Nicola**
**7750 E. Roseland**
**La Jolla California 92037 (US)**
Inventor: **Sutcliffe, J. Gregor**
**1467 Kings Cross Drive**
**Cardiff California 92007 (US)**
Inventor: **Shinnick, Thomas Michael**
**11738 Jonny Lane**
**San Diego California 92126 (US)**

(74) Representative: **Fisher, Adrian John et al**
**CARPMAELS & RANSFORD 43 Bloomsbury Square**
**London WC1A 2RA (GB)**

(56) References cited:
**Chemical Abstracts vol. 85, no. 8, October 1976 Columbus, Ohio, USA R. ARNON et al. "Immunological crossreactivity of antibodies to a synthetic undecapeptide analogues to the amino terminal segment of carcinoembryonic antigen, with the intact protein and with human serums" page 460, abstract no. 121543t**

(56) References cited:

Chemical Abstracts vol. 94, no. 17 27 April 1981
Columbus, Ohio, USA R.A. LERNER et al.
"Antibodies in chemically synthesized peptides
predicted from DNA sequences as probes of
gene expression" page 576, abstract no.
137530h

Chemical Abstracts vol. 88, no. 5, 30 January
1978 Columbus, Ohio, USA K. ITAKURA et al.
"Expression in Escherichia coli of a chemically
synthesized gene for the hormone
somatostatin" page 241, abstract no. 34407v

Chemical Abstracts vol. 73, no. 25 21 December
1970 Columbus, Ohio, USA M. SELA "Molecular
aspects of antigenicity" page 198, abstract no.
128947p

Chemical Abstracts vol. 73, no. 1 6 July 1970
Columbus, Ohio, USA M. SELA "Structure and
specificity of synthetic polypeptide antigens
page 168, abstract no. 1961h

Sutcliff et al., Nature, Vol. 287, No. 5785, pp.
801-805 (1980)

Arnon et al., Proc.Natl.Acad.Sci., 68(7), 1450-
1455 (1971)

Arnon, "Synthetic Vaccines - A Dream or
Reality", Advances in Experimental Medicine
and Biology, Proceedings of the Biological
Conference on New Concepts in Immunity of
Viral and Rickettsiae Diseases, ed. H.A. Kohn
and M.A. Klinberg, Plenum Press, 31: 209-222
(1972)

Zuckerman, Nature, 255: 104-105 (1975)
Langbeheim et al., Proc. Natl. Acad. Sci.,
73:4636-4640 (1976)

Adam et al., Biochem. Biophys. Res. Comm., 84:
677-683 (1978)

Wilhelm et al., Zbl. Bakt. Hyg. I. Abt Orig., B 166
18: 264-271 (1978)

## Description

The present invention relates to the production of novel synthetic antigens based upon information derived by DNA sequences and to the use of these antigens in the production of vaccines, diagnostic reagents, and the like. More specifically, this invention relates to such antigens formed by coupling to a carrier a synthetic antigenic determinant which immunologically corresponds to a portion of a natural protein (antigen).

In the prior art, antigens have been obtained in several fashions, including derivation from. natural materials, coupling of a hapten to a carrier, and by recombinant DNA technology. Sela et al (US—A—4 075 194, *Proc. Nat. Acad. Sci.,* U.S.A., Vol. 68, No. 7, pp. 1450—1455, July 1971; *Science,* Vol. 166, pp. 1365—1374, December 1969; *Adv. Immun.,* Vol. 5, pp. 29—129, 1966) have also described certain synthetic antigens.

Antigens derived from natural materials are the countless number of known antigens which occur naturally, such as blood group antigens, HLA antigens, differentiation antigens, viral and bacterial antigens, and the like. Considerable effort has been expended over the last century in identifying and studying these antigens.

Certain "synthetic" antigens have been prepared by coupling small molecules (such as, for example, dinitrophenol) to carrier molecules (such as, for example, bovine serum albumin), thus producing antigens which will cause production of an antibody to the coupled small molecule. The carrier molecule is necessary because the small moelcule itself would not be "recognized" by the immune system of the animal into which it was injected. This technique has also been employed in isolated instances to prepare antigens by coupling peptide fragments of known proteins to carriers, as described in the above-referenced Sela et al articles. For example, US—A—4 075 194 discloses the synthesis of an eleven amino acid sequence of carcinoembryonic antigen, and the coupling thereof to a carrier.

While this hapten-carrier technique has served the research community well in its investigations of the nature of the immune response, it has not been of significant use to produce antigens which would play a role in diagnostic or therapeutic modalities. The reasons for this dificiency are several.

First, to choose and construct a useful antigenic determinant from a pathogen (e.g., heptatis B virus) by this techique, one must determine the entire protein sequence of the pathogen to have a reasonable chance of success. Because of the difficulty of this task it has rarely, if ever, been done.

Second, even if one were willing to determine the entire protein sequence of a pathogen, other problems pertain. Specifically, many antigens of interest are expressed infrequently or not at all in the usual pathogen populations. For example, gonococcus expresses its pathogenic antigenic determinant only when in direct contact with host cells. Thus, the conventional approach of culturing the gonococcus *in vitro* may not yield the antigen of interest. Similarly, the oncogenic proteins of the known RNA and DNA viruses are not present in viral particles, but are expressed only when the virus interacts with the host cell. Consequently, one cannot obtain these proteins by the conventional techniques of virus isolation and protein purification.

Classically, vaccines are manufactured by introducing killed or attenuated organisms into the host along with suitable adjuvants to initiate the normal immune response to the organisms.while, desirably, avoiding the pathogenic effects of the organism in the host. The approach suffers from the well known limitations in that it is rarely possible to avoid the pathogenic response because of the complexity of the vaccine which includes not only the antigenic determinant of interest but many related and unrelated deleterious materials, any number of which may, in some or all individuals, induce an undesirable reaction in the host. For example, vaccines produced in the classical way may include completing antigens which are detrimental to the desired immune respnse, antigens which include unrelated immune response, nucleic acids from the organism or culture, endotoxins and constituents of unknown composition and source. These vaccines, generated from complex materials, inherently have a relatively high probability of inducing competing autoimmune responses even from the antigen of interest.

Recombinant DNA technology has opened new approaches to vaccine technology which does have the advantage that the manufacture begins with a single, pulse gene; however, much of this advantage is lost in actual production of antigen in *E. coli,* or other organisms. In this procedure, the gene introduced into a plasmid which is then introduced into *E. coli* which produces the desired protein, along with other products of the metabolism, all in mixture with the nutrient. This approach is complicated by the uncertainty that the desired protein will be expressed in the *E. coli* products. This problem is exhibited in the difficulty in manufacture of interferon. Further, even though the desired protein may be produced, there is uncertainty as to whether or not it can be harvested or whether it will be destroyed in the process of *E. coli* growth. It is well known, for example, that foreign or altered proteins are digested by *E. coli.* Even if the protein is present in sufficient quantities to be of interest, it must still be separated from all of the other products of the *E. coli* metabolism, including such deleterious substances as undesired proteins, endotoxins, nucleic acids, genes and unknown or unpredictable substances. Finally, even if it were possible, or becomes possible through advanced, though necessarily very expensive, techniques, to separate the desired protein from all other products of the *E. coli* metabolism, the vaccine still comprises an entire protein which may include undesirable antigenic determinants, some of which are known to initiate very serious autoimmune responses. Indeed, it is known that certain proteins which could otherwise be considered as vaccines include an antigenic determinant which induce such serious cross reference or side reactions as to prevent the use of the material as a vaccine. For example, a vaccine grown through the recombinant DNA technique just described from cloned Streptococci would result in a protein which could include an antigenic determinant which may cause heart disease, as well as the antigenic determinant of interest. Such a material would have little, if any, therapeutic potential as a vaccine.

Patent Application EP—A2—0 001 930 discloses a method of preparing peptides by use of an intervening microbial host. That application teaches that a synthetic DNA molecule is prepared whose sequence corresponds to a DNA sequence of a desired polypeptide. Start and stop codons are also synthesized and added appropriately to the synthetic DNA sequence. The synthetic DNA is thereafter placed in a plasmid, and the plasmid replicated in a microbe such as *E. coli.* The microbe produces the peptide fused to a microbial protein from which the desired peptide may be thereafter cleaved.

It is also possible, using hybridoma technology, to produce antibodies to viral gene products. Basically, hybridoma technology allows one to begin with a complex mixture of antigens and to produce specific antibodies later in the process.

In contrast, the present invention is the reverse process, in that we start with the ultimate in high purity antigenic determinant and thus avoid the necessity for purification of the desired antigenic product.

Hybridoma antibodies are known to be of low avidity and low binding constant, and therefore, have limited value.

Ultimately, in hybridoma technology, one must rely on the production of the antibody by cells which are malignant, with all of the attendant concerns regarding separation techniques, purity and safety.

Hybridoma production relies upon tissue culture or introduction into mice, with the obvious result that production is costly and there is inherent variability from lot to lot.

In addition, it is difficult to make a hybrid to molecules which comprise only a small percentage of the complex mixture one must start with.

That the process described below was successful in generating antibodies which reacted with native proteins of the murine leukemia and heptatis a viruses was actually quite surprising and, in fact, contrary to then current thinking in molecular biology. The surprise was twofold. First, the experiment involved a cascade of contingent events, each of which had to be successfully executed by few of which were routine. Many of those steps were necessary in generating the DNA sequences which were the blueprints for the peptide synthesis. That so many consecutive steps, each subject to vagaries imposed by their biological nature, could be carried out was a suprise. Second, that short linear peptide chains could elicit antibody responses in animals and that the elicited antibodies would recognize the much larger and more complex native structures of the proteins was quite a surprise.

As to the former point, the following steps had to be performed, and the following considerations rendered the likelihood of success at the end small if not remote. First, in cloning a cDNA copy of an RNA molecule, the retroviral enzyme reverse transcriptase is used to polymerize nucleotides complementary to the starting DNA. The fidelity of such a copying event is such that about one nucleotide in five hundred is miscopied. Furthermore, since that starting RNA is copied in the living cell from its gene by the enzyme RNA polymerase, another reasonably inaccurate replicating enzyme, another source of error is introduced. The cDNA copy is then linked to a plasmid vector, a process which has been shown often to involve rearrangments in the cloned DNA fragment. The plasmid is then introduced into a bacterium, and transformed colonies carrying the recombinant plasmid are selected. A colony of transformed bacteria is fragmented by streaking on a growth plate and a single isolate is picked for mass scale growth and DNA preparation. Since many rounds of replication have occurred in this process, there is no guarantee that one or more nucleotide altering events will not have damaged the gene, for which there has been no selective pressure throughout its isolation. Such damage could render the DNA sequence of such a gene meaningless or greatly lessen its utility in acting as a blueprint for selection of peptides and their synthesis. The obstacles summarized thus far are those involved in isolating DNA for sequence analysis such that the DNA is exactly representative of the original virus gene.

The next set of operations lead to a DNA sequence of the virus gene. At the onset of these studies, the prospect of the scientist being able to solve accurately gene-sized nucleotide sequences in a routine manner was just emerging. In 1977, Sanger and coworkers (Sanger et al, 1977, *Nature* 265: 687) described their efforts to solve the nucleotide sequence of the small bacterial virus ΦX174. Their solution, which contained more than 30 errors in 5300 nucleotides, relied heavily on protein and RNA sequencing to interpret the DNA sequencing results and occupied more than ten professional scientists for some five years. These authors, one of whom was awarded the Nobel Prize for the work, concluded that "As with other methods of sequencing nucleic acids, the plus and minus technique used by itself cannot be regarded as a completely reliable system and occasionally errors may occur. Such errors and uncertainties can only be eliminated by more laborious experiments and, although much of the sequence has been so confirmed, it would probably be a long time before the complete sequence could be established. We are not certain that there is any scientific justification for establishing every detail . . .". A protein sequence predicted from a solved DNA sequence of a gene thus remains hypothetical until the proof of its accuracy emerges. Such proofs are now of three forms — either the partial amino acid sequence analysis of the gene's protein product, or the observation that antibodies to synthetic peptides from within the protein predicted by the

4

DNA sequence react with the native protein, or actual expression of the functional protein by the cloned gene (a relatively rare event).

Previous studies by Arnon et al. (1971, *Proc. Nat. Acad. Sci.* 68: 1450), Atassi (1975, *Immunochemistry* 12:423) and Vyas et a. (1972 *Science* 178:1300) have been interpreted by these authors to indicate that short linear amino acid sequences are, in general, unlikely to elicit antibodies reactive with the native protein structure. It was thought that for most regions of most molecules, antigenic determinants resulted from amino acid residues well separated in the linear sequence but close together in the folded protein. The exact three dimensional conformation of the peptides used to elicit antibodies was thought to be critical in most cases, even for those antigens involving amino acids close together in a sequence. For example, Sela thought it necessary to synthesize a rather elaborate loop structure to elicit an anti-lysozyme response; Atassi engineered many elaborate molecules, each intended to mimic the tertiary structure of the target protein, and Vyas concluded that the hepatitis B surface antigen's three dimensional conformation was a critical factor in eliciting antibodies reactive with that native structure. The discovery claimed herein proves that the concerns which made others expect that these experiments would not work were unwarranted. We have discovered, however, that antibodies to linear peptides react with native molecules and elaborate syntheses are unnecessary, uneconomical and obsolete in view of the step forward represented by the present invention.

The present invention provides a chemically synthesized antigenic peptide containing an amino acid sequence substantially corresponding to an antigenic amino acid sequence present in a natural, pathogen related protein, said peptide when linked to a carrier and introduced into a host animal being capable of eliciting the production of antibodies that react with said natural pathogen related protein, and said peptide, taken from left to right and in the direction from amino-terminus to carboxy-terminus, being represented by a formula selected from the group consisting of:

(a) LeuThrGlnGlnPheHisGlnLeuLysProIleGluCysGluPro;

(b) IleLeuAsnArgLeuValGlnPheValLysAspArgIleSerValValGlnAlaLeuValLeuThr GlnGlnPheHisGlnLeuLysProIleGluCysGluPro;

(c) PheProGlySerSerThrThrSerThrGlyProCysArgThrCysMetThrThrAlaGlnGlyThr SerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValPro AsnGlyThrLeuValLysThrIleThrAsnAspGlnIleGlu;

(f) AsnAlaThrGluLeuValGlnSerSerSerThrGlyLysIleCysAsnAsnProHisArgIleLeu AspGlyIleAsnCys;

(g) CysAsnAsnProHisArgIleLeuAspGlyIleAsnCysThrLeuIleAspAlaLeuLeuGlyAsp ProHisCysAspGlyPheGlnAsnGluLysTrpAspLeuPhe;

(h) AspTyrAlaSerLeuArgSerLeuValAlaSerSerGlyThrLeuGluPheIleAsnGluGlyPhe AsnTrpThrGlyValThrGlnAsnGlyGlySerSerAlaCys;

(i) SerGlyLysValThrValSerThrLysArgSerGlnGlnThrIleIleProAsnValGlySerArg ProTrpValArgGlyLeu;

(j) CysProLysTyrValLysGlnAsnThrLeuLysLeuAlaThrGlyMetArgAsnValProGluLys GlnThrArg;

(k) GlnAspLeuProGlyAsnAspAsnAsnSerThrAlaThrLeuCys;

(l) CysLeuGlyHisHisAlaValProAsnGlyThrLeuValLysThrIleThrAsnAspGlnIleGlu ValThrAsnAlaThrGluLeuValGlnSerSerSerThrGlyLysIleCys;

(m) CysAsnAsnProHisArgIleLeuAspGlyIleAsnCys;

(n) CysAsnAsnProHisArgIleLeu;

(o) HisCysAspGlyPheGlnAsnGluLysTrpAspLeuPheValGlu;

(p) HisCysAspGlPheGlnAsnGluLysTrpAspLeuPheValGluArgSerLysAlaPheSerAsn CysTyrProTyrAspValProAspTyrAlaSerLeuArgSer;

(q) ValThrGlnAsnGlyGlySerSerAlaCysLysArgGlyProAspSer;

(r) CysLysArgGlyProAspSerLysArgGlyProAspSerGlyPhePheSerArgLeuAsnTrpLeuTyr;

(s) CysLysArgGlyProAspSerLysArgGlyProAspSerGlyPhePheSerArgLeuAsnTrpLeu TyrLysSerGlySerTypTyrProValGlnAsnValTrpMetProAsnAsnAsp AsnSer;

(t) AsnSerAspLysLeuTyrIleTrpGlyValHisHisProSerThrAspLysGluGlnThrAsnLeu TyrVal;

(u) HisHisProSerThrAspLysGluGlnThrAsnLeuTyrVal;

(z) CysLeuGlyGlnAsnSerGlnSerProThrSerAsnHisSerProThrSerCysProProThrCys ProGlyTyrArgTrpMetCysLeuArgArgPheIle;

(aa) GluAsnIleThrSerGlyPheLeuGlyProLeuLeuValLeuGln;

(bb) LeuThrArgIleLeuThrIleProGlnSerLeuAspSerTrp;

(ee) LeuValLeuLeuAspTyrGlnGlyMetLeuProValCysProLeu; and

(ff) any peptide comprising at least a sequence of 6 or more amino acids selected from the sequence of (a) through (ee) above.

According to the present invention, the proportion of protein of interest in the organism is not of particular importance because one may start with the gene which generates the protein and all genes are generally present in more or less equal quantities. Thus, one may manufacture antigens to induce an antibody response to even minor protein constituents in an organism.

Since, in the present invention, one obtains an antigen which is free of all competing and interfering proteins, the vaccine produces protective or recoverable antibodies which are specific to the antigenic determinant of interest; hence, there is no cross reactivity with any other antigenic determinant.

Since the antibodies of the present invention can be grown in virtually any host of interest, in large animals such as sheep, equine animals, etc., one may very easily and inexpensively provide a long-life source or "pool" of antibodies of virtually perfect identity and reliability simply by periodically harvesting antibodies from the same host. This is a great advance over the cost and lack of reliability of growing antibodies as taught in the prior art.

It is possible by the principles of the present invention to design and produce antigen for use as a protective vaccine or to produce antibodies for any desired purpose or use which totally overcomes the very serious autoimmune response to many antigens which has prevented or severely limited the use of whole protein antibodies in therapy and diagnosis. For example, one can easily produce a vaccine which includes only the specific antigenic determinant which initiates the production of antibodies which bind the antigenic protein, free of cross referencing determinants which create very serious side reactions, e.g., heart disease in the case of Streptococci and the serious neurologically manifested autoimmune reaction to the recent massive "swnie flue" vaccination, with its sometimes catastrophic consequences.

In summary, all of the prior art methods, including the recent recombinant DNA and hybridoma methods, of manufacturing vaccines and antibodies are, as compared with the present invention, more complicated and expensive technically, more time consuming, are low yield quantitatively and qualitatively, and all present some level of concern as to safety and reliability inherent in any method in which the desired component must be separated from undesired components. Further, the prior art methods do not provide a route to the production of certain vaccines or to the elimination of autoimmunological reactions of a given antigen, whereas the present invention opens the way to these new results. The present invention offers new, unpredicted results in a simpler, less expensive and more direct and productive way than contemplated by the prior art.

The present invention overcomes these and other barriers and disadvantages in prior art methods of producing novel synthetic antigens. These antigens may be used not only to produce vaccines and diagnostic antibodies, but also for producing cell mediated immune response, high titre antibody for passive immunoprophylaxis, and for other purposes as will be clear from the present disclosure. The vaccines of this invention are totally free of viral genomes, bacterial nucleic acids, and endotoxins. The vaccines and antibodies of this invention are specific to the desired antigen and do not cross reference to spurious antigenic determinants which may appear on nonpertinent portions of the antigen.

As a vaccine, the present invention comprises an antigen carrier, which may be of any of numerous recognized carriers, to which a synthetic peptide which corresponds to an antigenic determinant portion of a natural protein is bound, the peptide functioning as the specific antigenic determinant of the resulting antigen, which, when the antigen is introduced into the desired host, initiates the production of antibodies or a cell mediated response in the host to the aforesaid antigenic determinant portion of the natural protein.

Very significantly, as will be discussed hereafter, the invention also contemplates antigens in which the entire carrier is antigenic.

The synthetic antigen formed by coupling the synthetic antigenic determinant unit to an antigen carrier, and the method of preparing this synthetic antigen are specific aspects of the present invention.

In general, the synthetic antigen (which corresponds immunologically to the natural antigen containing the specific antigenic determinant) may be formed by the steps of:

(a) determining from a genome (either DNA or RNA) the amino acid sequence of all or part of a peptide region of interest;

(b) predicting regions of the peptide which are potential antigenic determinants;

(c) preparing a synthetic antigenic determinant peptide which immunologically duplicates onoe or more of the antigenic determinants of the peptide which correspond to a natural antigen; and, if necessary,

(d) coupling the synthetic determinant produced in step (c) to a pharmaceutically acceptable carrier, whereby the desired synthetic antigen is prepared.

As a method of manufacturing vaccines, the method comprises the steps of determining from the DNA sequence (or small cDNA sequence if the genome is RNA) of the organism in question of amino acid sequence of an antigenic determinant portion of a protein antigen, synthesizing a peptide which antigenically is the duplicate or substantial duplicate of the determinant portion of the protein, and, if necessary, attaching the synthetic peptide to a carrier or forming a carrier to form an antigen in which the peptide is the specific antigenic determinant and which, when introduced into a host, initiates production of antibodies to the protein antigen.

As a method of manufacturing antibodies, the vaccine as described above, is injected into a host and antibodies to the protein antigen are harvested from host fluids for use in conventional diagnostic procedures to detect the presence of the protein antibody or as therapeutic agents for passive immunoprophylaxis.

It is possible to manufacture vaccines to induce protective antibody production or a cell mediated immune response in a host against pathogenic agents which do not themselves express the antigenic determinant of interest. For example, in RNA tumor virus the proteins responsible for cell transformation while specified in the genetic materials are not expressed in the viral particle per se. Therefore, vaccine production from killed or attenuated virus is not possible. A similar situation occurs in viral induced feline leukemias and lymphoma. This result was never before capable of accomplishment.

It will be understood that while there are many procedural steps utilizing many materials in the manufacture of the vaccines and antibody preparations of this invention and in carrying out the methods of this invention, as discussed in detail hereinafter, the invention is conceptually as stated above and as defined with particularity in the claims appended hereto.

In a general sense, then, one aspect of the invention is a general process for producing vaccines which have all of the immunizing effect of prior art vaccines but which are totally free of competing or cross referencing immunological side effects.

The invention is also a general process for manufacturing vaccines to organisms which do not themselves contain the antigenic determinant.

Vaccines which include known immunologically significant antigenic determinants which are in nature associated in a protein with a determinant which initiates an adverse reaction, such as an autoimmune reaction, but which are free of the adverse determinant are also manufactured according to the methods of this invention.

Vaccines to viral diseases which are specific to a specific antigenic determinant of the virus or induced by the virus in the host are important aspects, features and products included in this invention, but the invention encompasses vaccines which are particular to specific antigenic determinants in or induced by such eukaryotes and prokayotes as bacteria, fungus, somatic cells and yeasts, all of which are of equal importance in their own sphere of action to the viral vaccines.

Antibody preparations, for diagnosis, inducing temporary immunity, and other uses produced by the immune response of a host to the above described vaccines and harvested in the traditional ways are also important in the general concept and specific applications of this invention.

Vaccines are manufactured by identifying a peptide sequence, chemically synthesizing the peptide sequence, and attaching the resulting synthetic peptide to a carrier thus forming an antigen, and, of course, establishing by immunochemical techniques the initiation of protective or harvestable antibodies in the host system or the initiation of a cell mediated immune response. The peptide sequence may be any oligopeptide which includes an antigenic determinant which is specific to the pathogen or organism under consideration, whether contained in the organism or induced by the organism or pathogen in the host. Antibody preparations, vaccines and the method of preparing the same all constitute integral and important facets of this invention, as does the synthetic antigen and its method of preparation.

The invention comprises a number of facets, all related to the production, composition, and use of synthetic peptide specific antigenic determinants. Various of these facets are enumerated below but without effort to encompass each and every significant facet of the invention in this summary.

The invention contemplates manufacturing antigens by identifying the portion of a genome of an organism which encodes a potential antigenic peptide, cloning the gene thus identified and determining the nucleotide sequence of a cloned gene. The amino acid sequence of the antigen peptide is solved from the nucleotide sequence of the gene and the thus determined amino acid sequence is chemically synthesized into at least one peptide which is then attached to an antigen carrier or made part of an antigen and introduced into a host. Antibodies, the production of which are initiated by the introduction of the antigen into the host, are immunologically screened to determine which antigen induces antibody to the organism whose genomme was used to originate the peptide sequence. Once the antigen is shown to produce antibodies to the naturally occurring antigen in the organism, then the antigenic peptide is manufactured in sufficient quantities to be used, along with or as part of a carrier, as a vaccine against pathological invasion by the organism. In general, the peptide must have a minimum of four to six, usually six, amino acids, and often as many as eight amino acids are required to initiate the production of antibodies to a corresponding naturally occurring antigen. Peptides having about fifteen or more amino acids in the sequence are preferred and provide a much higher degree of specify and antigenic response.

Antibody compositions which may be used in therapeutic or diagnostic applications are manufactured using the technique described before, but with the further step that the antibodies produced to the synthetic antigen described are harvested from the host.

As a method for preparing antigen which includes a peptide antigenic region and the carrier, the structure of the peptide antigenic region is determined by mapping the genome that directs the production of at least one antigenic determinant of a naturally occurring antigen and then determining from the genome nucleotide sequence (map) the sequence of the peptide coded for by said genome, followed by chemically synthesizing the peptide antigenic region whose sequence was thus determined and forming an antigen from this antigenic region.

Antigens manufactured according to this invention consist essentially of the chemically synthesized peptide antigenic region and a carrier; the peptide having a sequence which was determined from the genome which directs the production of at least an antigenic determinant of a naturally occurring antigen, the antigen thus synthesized being free of naturally occurring proteins and protein fragments, the synthetic peptide antigenic region substantially duplicative of a naturally occurring antigenic determinant and capable of initiating production of antibodies to said naturally occurring antigenic determinant when injected into a host organism.

Antigen, consisting essentially of a carrier portion and a chemically synthesized peptide portion which is substantially duplicative of a naturally occurring antigenic region of a protein which induces an immunological response in the host infected with an organism in which such protein is not expressed, is prepared by determining the sequence of the peptide from the genome of the organism which directs the production of the naturally occurring antigenic region. Antigens of this type are, of course, not capable of being produced from the organism itself. Antibodies may be grown in a host in response to the introduction of such antigens into the host followed by harvesting of the antibodies for therapeutic or diagnostic purposes.

A general procedure is disclosed for preparing antigen compositions, which consists essentially of a synthetic peptic antigenic determinant region and a carrier portion, by determining from the genome of an organism the region of the peptide coded for by this genome and those regions of said peptide which are likely to have specific antigenic determinant portions therein, or to constitute the same, chemically synthesizing at least one of these regions of the peptide, forming a potential antigen from this synthesized region of the peptide and injecting the potential antigen into a host, followed by determining if the potential antigen induces in the host antibodies to the organism from whose genome the peptide sequence was derived and then manufacturing antigen from chemically synthesized peptide regions which are shown by the preceding steps to induce in the host antibodies to naturally occurring specific antigenic determinants of said organism.

In another facet, the invention contemplates manufacturing antigens by forming poly(peptide fragment) polymers or copolymers by linking together a plurality of chemically synthesized peptide regions at least several of which were shown to induce in the host antibodies to the organism. It is possible, using this approach, to manufacture antigens in which all, or at least a substantial portion, of the antigen constitutes specific antigenic determinant regions. All these antigenic determinant regions may be identical, or they may be alternating, for example, two or more antigenic determinants may be synthesized or copolymerized together, giving an antigen having a number of specific antigenic determinant regions different from one another. Non-antigenic peptide regions may be included within the antigen, if desired, and it is contemplated that the invention would encompass at least some non-antigenic peptide sequence in such antigens, although this would not be required. This approach even opens the path to providing a single antigen which may, for example, immunize against more than one organism. For example, if an antigen is synthesized of a synthetic peptide specific antigenic determinant for organism A and a different synthetic peptide specific antigenic determinant for organism B, then an antigen which will induce the production of antibodies to both organism A and organism B is included in a single antigenic structure, free antigen, free of all proteins and protein fragments which result from other methods of antigen production. A new antigen, free or substantially free of portions which function only as carrier, and which consists essentially of one or a plurality of synthetic peptide specific antigen determinant regions polymerized or copolymerized together, at least some of which regions are substantially duplicative of naturally occurring specific antigen determinant regions, and inducing, when introduced into a host, antibodies to naturally occurring specific antigenic determinant regions, is manufactured according to the principles of this invention. No such antigen has ever been manufactured before.

The invention also contemplates specific antigens of defined amino acid sequence synthetic peptides. For example, specific antigens for initiating the production of antibodies to hepatitis B and influenza are identified and claimed, among other specific antigenic determinants. In this regard, it is to be understood that, as in all or nearly all antigenic determinant regions, especially long antigenic determinant regions, certain amino acids may be replaced with other amino acids without destroying the antigenic character of the peptide and are fully equivalent to the specifically named peptide. Such specific antigenic determinants are regarded as equivalent for the purpose of this invention and are included and contemplated when specific peptide sequences are referred to or defined.

In yet another facet, the invention contemplates a lysosome consisting essentially of a lipd rich nucleus portion having at least one synthetic peptide specific antigenic determinant attached thereto by means of a lipid moiety linked to the peptide, the synthetic peptide specific antigenic determinants substantially duplicating a naturally occurring antigenic determinant. Such lysosomes may have many identical or many different synthetic peptide specific antigenic determinants attached thereto in the same manner.

The various facets of the invention may be utilized in forming a single antigen or antibody or other peptide containing product in which the specific antigenic determinant feature of the peptide is the significant, or at least a major, factor insofar as the biological function of the product is concerned. For example, liposomes may include fatty acid-peptide moieties in which the peptide portion includes two or more specific antigenic determinant regions specific to two or more organisms by mimicking a naturally occurring specific antigenic determinant of the organisms of interest. Integral antigenic carriers formed of a plurality of synthetic peptide specific antigenic determinants may be synthesized end-to-end, linked after synthesis, or prepared by a combination of these approaches. Other combinations are also contemplated.

In the accompanying drawings:

Figure 1 depicts the nucleotide sequence of the 3' end of the Mo-MuLV provirus.

# EP 0 044 710 B2

Figure 2 depicts an autoradiograph of an SDS-PAGE separation of labelled SCRF 60A cell lystate reacted with various antisera including antisera to the new synthetic vaccine of this invention.

Figure 3 is a revised genetic map for Mo-MuLV provirus.

Figure 4 depicts an autoradiograph of SDS-PAGE separation of labelled lystate of SCRF 60A cells referred to in Figure 2.

Figure 5 shows a comparison of portions of the genetic maps of Mo-MuLV and AKV viruses.

Figure 6 is a scanning electron photomicrograph of the surface of a virus particle decorated by antibodies grown against the R-synthetic antigen example of this invention and conjugated with ferritin for visualization.

Figure 7 depicts peptide sequence and position (corresponding to the underlined residues in Figure 8). the underlined residues were not in the primary protein sequence but were added to allow coupling to carrier or radioiodination. All peptides except peptides 1, 3a and 7 were coupled to carrier protein KLH as described in Methods. Peptide 1 was used without coupling to KLH. Peptides 3a and 7 were insoluble and not used.

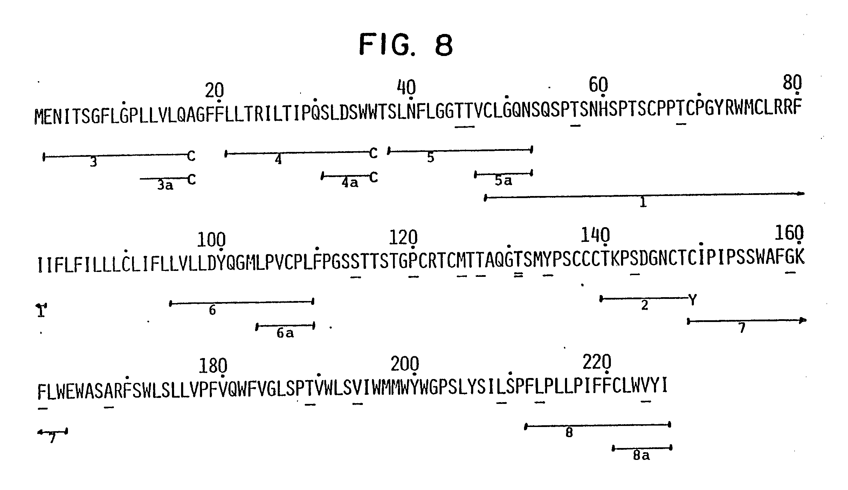

Figure 8 depicts that the 226 amino acid sequence of $HB_sAg$ as translated by Pasek et al. from the nucleic acid sequence is presented in one letter code (A ala, C cys, D asp, E glu, F phe, G gly, H his, I ile, K lys, L leu, M met, N asn, P pro, Q gln, R arg, S ser, T thr, V val, W trp, Y tyr) to indicate which regions of the protein were chosen for synthesis. The boldly underlined regions which correspond to those peptides are numbered 1—8 or 3a—6a, 8a. C or Y at the end of a bold underline indicates where a cysteine or tyrosine not found in the primary sequence was added for technical reasons. Residues which are not the same in all three nucleotide sequence determinations are lightly underlined. Many of these cluster between 110—140.

Figure 9 is an autoradiographic photo of radioactively labelled, purified Dane particles which were reacted with 5 µl of normal, anti-peptide 3 or anti-peptide 4 serum. Precipitates were collected and prepared for electrophoresis as described in Methods. Samples were electrophoresed on a 5—17% SDS-polyacrylamide gel and autoradiographed.

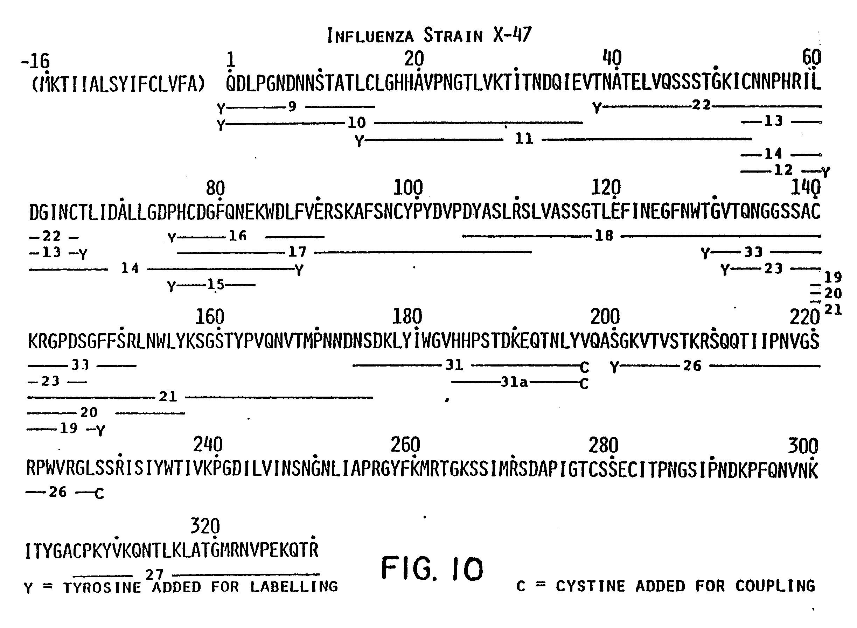

Figure 10 depicts the genome and translation of the genome of the Influenza X-47 strain and potential specific antigenic regions. Cystine is added to the peptide region which is considered a potential antigenic determinant for coupling and tyrosine is added to attach a label, e.g. a radiolabel, to the peptide.

Figure 11 depicts a poly(specific antigenic determinant peptide) copolymer antigen.

Methods and materials unique to this invention are described hereinafter with the particular procedure under consideration. In general, however, specific laboratory techiques, methods and materials are those used in molecular biology and biochemistry generally.

Particular reference is made to *Methods in Enzymology,* Colowick, S. P. and Kaplan, N. O., Editors, Academic Press, New York; *Methods in Immunology and Immunochemistry,* Academic Press, and *Handbook of Biochemistry and Molecular Biology,* Chemical Rubber Publishing Company, for a description of a reference to the general materials and techiques of interest.

The manufacture of haptens and antigens by attachment of a determinant to a carrier is a very well known technique and numerous carriers have been described. Commonly known carriers include keyhole limpet hemocyanin (KLH), bovine serum albumin (BSA), sheep erythrocytes (SRBC), D-glutamic acid:D-lysine.

No single laboratory technique is, per se, novel; rather, the invention resides in the products which never before existed and which constitute a large step functional advance over the nearest prior art products, and to the processes as a whole for preparing the products of this invention. The following references are provided as background for these procedures:

References
1. Baltimore, D., Cold Spring Harbor Symp., *Quant Biol. 39,* 1187—1200 (1974).
2. Oskarsson, M. K., Elder, J. H., Gautsch, J. W., Lerner, R. A. and Vande Woude, G. F., *Proc. Natl. Acad. Sci.,* U.S.A. 75, 4694—4698 (1978).
3. Gautsch, J. W., Elder, J. H., Schindler, J., Jensen, F. C., and Lerner, R. A., *Proc. Natl. Acad. Sci.,* U.S.A. 75, 4170—4174 (1978).
4. Jamjoon, G. A., Naso, R. B. and Arlinghaus, R. B., *Virol. 78,* 11—34 (1977).
5. Famulari, N. C., Buchhagen, D. L., Klenk, H. D., and Fleissner, E., *J. Virol. 20,* 501—508 (1976).
6. Witte, O. N., Tsukamoto-Adey, A. and Weissman, L. L., *Virol. 76,* 539—553 (1977).
7. Fan, H. and Verma, I. M., *J. Virol. 26,* 468—478 (1978).
8. Sutcliffe, J. G., Shinnick, T. M., Lerner, R. A., Johnson, P. and Verma, I. M., Cold Spring Harbor Symp. *Quant. Biol. 44,* in press (1979).
9. Sutcliffe, J. G., Shinnick, T. M., Verma, I. M. and Lerner, R. A., *Proc. Natl. Acad. Sci.,* U.S.A. in press (1980).
10. Marglin, A. and Merrifield, R. B., *Ann. Rev. Biochem. 39,* 841—866 (1970).
11. Pederson, F. S. and Haseltine, W. A., *J. Virol. 33,* 349—365 (1980).
12. *Atlas of Protein Sequence and Structure,* Vol. 5, Sup. 3, M. O. Dayhoff, ed., Natl. Biomed. Res. Found., pub. Washington, D.C. (1978).
13. Dayhoff, M. O., Schwartz, R. M. and Orcutt, B. C., pp. 352, op. cit.
14. Fisher, R. A., *The Genetical Theory of Natural Selection,* Clarendon Press, Oxford (1930).

9

15. Elder, J. H., Gautsch, J. W., Jensen, F. C., Lerner, R. A., Harley, J. W. and Rowe, W. P., *Proc. Natl. Acad. Sci.,* U.S.A. 74, 4676—4680 (1977).

16. Lerner, R. A., Jensen, F. C., Kennel, S. J., Dixon, F. J., Roches, G. D. and Francke, U., *Proc. Natl. Acad. Sci.,* U.S.A. 69, 2965—2969 (1972).

17. Niman, H. L. and Elder, J. H., *Proc. Nat. Acad. Sci.,* U.S.A., in press (1980).

18. Edwards, S. A. and Fan, H., *J. Virol, 30,* 551—563 (1979).

19. Kitagawa, T. and Ailawa, T., *J. Biochem. (Tokyo)* 79, 233 (1976).

20. Liu, F., Zinnecker, M., Hamaoka, T. and Katz, D. H. *Biochem. 18,* 690 (1979).

21. Katz, David H., U.S. Patent No. 4,191,668, March 4, 1980.

22. *J. Exp. Med., 134:* 201—203 (1971).

23. *J. Exp. Med., 136:* 426—438, 1404—1429 (1972).

24. *J. Exp. Med., 138:* 312—317 (1973).

25. *J. Exp. Med., 139:* 1446—1463 (1974).

26. *Proc. Natl. Acad. Sci.,* U.S.A., 71: 3111—3114).

27. *Proc. Natl. Acad. Sci.,* U.S.A., 73: 2091—2095 (1976).

28. *J. Immunol. 114:* 872—876 (1975)).

29. *J. Immunol. 120:* 1824—1827 (1978).

30. *J. Exp. Med., 139:* 1464—1472 (1974).

31. Humphrey, J. H. and White, R. G., *Immunology for Students of Medicine,* Blackwell, Oxford (1970).

32. Katz, David H. and Benacerraf, Baruj, *Immunological Tolerance, Mechanisms and Potential Therapeutic Applications,* Academic Press (1974).

33. *Newsweek,* March 17, 1980, pp. 62—71.

34. *Chemical & Engineering News,* June 23, 1980, p. 10.

35. Milstein, C., *Differentiation 13:* 55 (1979).

36. Howard, J. C., Butcher, G. W., Galfre', G., Milstein, C. and Milstein, C. P., *Immunol. Rev. 47:* 139 (1979).

37. Hammerling, G. J. Hammerling, U., and Lemke, H., *Immunogenetics 8:* 433 (1978).

38. Shulman, M., Wilde, C. D., and Köhler, G., *Nature 276:* 269 (1978).

39. Köhler, G. and Milstein, G., *Nature 256:* 495 (1975).

40. Ledbetter, J. A. and Herzenberg, L. A., *Immunol. Rev. 47:* 63 (1979).

41. Gefter, M. L., Margulies, D. H. and Scharff, M. D., *Somatic Cell Genetics 3:* 231 (1977).

42. Köhler, G. and Milstein, C., *Eur. J. Immunol. 6:* 511 (1976).

43. *J. Biol. Chem., 241:* 2491—2495 (1966).

44. *J. Biol. Chem., 242:* 555—557 (1967).

45. Koprowski, Hilary et al, U.S. Patent No. 4,196,265, April 1980.

46. *Science 209,* No. 4463, pp. 1319—1438 (September 1980 — entire number).

47. Davis, B. D., Dulbecco, R., Eisen, H. N., Ginsberg, H. S., Wood, W. B. Jr., and McCarty, M., *Microbiology,* Harper & Row, Hagerstown, Md., 1973.

48. Morgan, J. and Whelan, W. J., *Recombinant DNA and Genetic Experimentation,* Pergamon Press, New York, 1979.

49. Goldstein, L. and Prescott, D. M., *Cell Biology, A Comprehensive Treatise,* Vols. 1, 2 & 3, Academic Press, San Francisco.

50. Scot, W. A. and Werner, R., *Molecular Cloning of Recombinant DNA,* Academic Press, New York 1977.

51. Wu. Ray (Ed.), Colowick, Sidney P., and Kaplan, Nathan O., *Methods in Enzymology,* generally and Vol. 68, "Recombinant DNA" in particular, Academic Press, New York.

52. Cooper, Terrance G., *The Tools of Biochemistry,* John Wiley & Sons, New York, 1977.

53. Sela, Michael, *Science 166:* 1365—1374 (1969).

54. Arnon, R., Elchanan, M., Sela, M. and Anfinsen, C. B., *Proc. Natl. Acad. Sci.* U.S.A., 68: 1450 (1971).

55. Sela, M., *Adv. Immun. 5:* 29—129 (1966).

56. Sela, M., Arnon, R., and Chaitchik, S., U.S. Patent No. 4,075,194, February 21, 1978.

57. Cohen, S. N., and Boyer, H. W., U.S. Patent No. 4,237,224, December 2, 1980.

58. Lerne, R. A., Sutcliffe, J. G. and Shinnick, T. M. (1981), *Cell 23:* 109—110.

59. Wilson, I. A., Skehel, J. J. and Wiley, D. C. (1981), *Nature 289:* 366—3763.

60. Sutcliffe, J. G., Shinnick, T. M., Green, N., Liu, F-T, Niman, H. L., and Lerner, R. A. (1980), *Nature 287:* 801—805.

By 1970, Marglin and Merrifield were able to report that "Our ability to synthesize peptides of intermediate size is now well established". (*Chemical Synthesis of Peptides and Proteins,* A. Marglin and R. B. Merrifield, *Ann. Rev. Biochem.* 39:841—866, at 862 (1970). The Merrifield et al synthesis was used in proving the practicality of the present invention; however, the techniques of synthesis per se is not critical.

DNA mapping, the general characterization of genomes, and the prediction of protein amino acid sequence from the genetic code are all very well established techniques.

A well-studied virus was selected as one vehicle to prove the present invention in an effort to avoid any possible ambiguity in results. It will be apparent, however, that this selection is not limiting in the least and that the invention is of general applicability to any system in which it is desired to prepare a vaccine to protect against any proteinaceous antigen which includes a peptide which is or includes a specific antigenic determinant for the antigen. Thus, the antigen of interest may be of viral, bacterial or fragments or somatic cells, provided only that a specific antigenic determinant peptide immunologically characterizes the antigen.

The techiques and materials for binding antigenic determinants to carriers is well known, and no particular novelty is attached to this individual step per se, but rather the invention lies in the creation of a monospecific antigen from a synthetic specific antigenic determinant peptide and a carrier to create a vaccine which obviates the problems and limitations which inhere in the prior art.

Many steps are taken and procedures are carried out during the inventive method to separate out the various materials and reagents, to identify components and to prove that the reactions and results sought have occurred.

Techniques of general usage are described in standard texts and treatises. Reference is made, for example, to *Methods in Enzymology, supra; Methods in Immunology and Immunochemistry, supra; Handbook of Biochemistry and Molecular Biology, supra;* Dyson, Robert D., *"Cell Biology — A Molecular Approach",* 2nd Ed., Allyn and Bacon, Boston (1978); Pelczar, Michael J., Jr., Reid, Roger D., Chan, E. C. S., *"Microbiology",* 4th Ed., McGraw-Hill (1977); Bohinkski, R. D., *"Modern Concepts in Biochemistry",* 2nd Ed., Allyn and Bacon, Boston (1976). Specific procedures are published (References 1—20, 21—30, 32, 35—43) and used without significant change.

The steps of the methods of the invention applicable in any given utilization of the invention depend upon the state of knowledge respecting and availability of specimens of the particular antigen of interest. It is convenient to consider the methods in stages.

I. Characterization and identification of antigenic determinant

If the antigenic determinant is known and the amino acid sequence of the peptide chain which includes or constitutes the specific antigenic determinant is known, then one would proceed immediately to manufacture a synthetic peptide which, immunologically, duplicates the specific antigenic determinant of the antigen of interest.

This approach may become more attractive in the future but presently it will be necessary, usually, to identify the peptide which is or includes the determinant and to determine the amino acid sequence of the peptide. The amino acid sequence may be determined directly or indirectly by DNA mapping of the genome which directs the production of the peptide portion of interest. The procedures used in this invention are known from a theoretical point of view, and DNA mapping of the genome which directs the production of the peptide portion of interest, is well enough defined now to become a practical tool. The determination of amino acids sequence directly from the protein is such an arduous and uncertain procedure as to be of little academic value and of virtually no practical value at this point in time. While direct amino acid sequence determination from proteins of peptides may one day become a practical tool, the presently available approaches are not such as to recommend this process for manufacturing operations or for the synthesis of synthetic peptide specific antigenic determinants. Thus, the starting point of the invention ultimately is the genome which determines the amino acid sequence of the peptide portion which contains the specific antigenic determinant regions.

Once the amino acid sequence is known or predicted, the peptide is synthesized using the Merrifield et al technique, or any other technique which may be or become available. In an abstract sense, one could proceed directly from the peptide synthesis to the vaccine but prudence, at least with the present state of knowledge, dictates that the thus synthesized peptide be shown unequivocally to be the same as or at least immunologically equivalent to the specific antigenic determinant of the antigen of interest. This proof is made through chemical and physical characterizations and, ultimately, by establishing an immune reaction between antibodies to the synthetic determinant and the natural antigen. Well known techniques for establishing molecular weights, charge, binding affinity, etc. are used in this characterization. Chapter 2 of *"Modern Concepts in Biochemistry" supra,* entitled "Methods of Biochemistry and the appendix "Tools of the Cell Biologist" to *"Cell Biology", supra,* describe these characterization procedures in general and cite complete descriptions of specific procedures and techniques. Radioimunoassay procedures are widely used and well described by Hunter, W. M. *"Preparation and Assessment of Radioactive Tracers", British Medical Bulletin,* 30:18—23. Specific examples of such characterizations are given hereinafter.

II. Manufacture of vaccine antigen

Once one has on hand the synthetic peptide which has been shown to be the specific antigenic determinant of the antigen of interest, than known binding techniques are utilized to bind the determinant to a carrier to form the antigen in situations where such a carrier is necessary. This step may, of course, be carried out on a small scale in the course of establishing the immunological identity of the peptide to the antigenic determinant. For example, in initially proving the invention in our laboratory, Dr. Fu-Tong Liu kindly aided by attaching the synthetic protein we had prepared to a known carrier, KLH, using published materials and techniques. (Liu, Fu-Tong et al, *"New Procedures for Preparation and Isolation of Conjugates of Proteins and a Synthetic Copolymer of D-Amino Acids and Immunochemical Characterization of such Conjugates", Biochemistry* 18: 690—697 (1979).

The choice of carrier is more dependent upon the ultimate intended use of the antigen than upon the determinant of the antigen, and is based upon criteria not particularly involved in the present invention. For example, if the vaccine is to be used in animals, a carrier which does not generate an untoward reaction in the particular animal will be selected. If the vaccine is to be used in man, then the overriding matters relate to lack of immunochemical or other side reaction of the carrier and/or the resulting antigen, safety and efficacy — the same considerations which apply to any vaccine intended for human use. In practice, it is contemplated that the present invention will find its first, wide applicability in animals, e.g. pets and farm animals.

III. The immune response — antibody manufacture

Upon injection, or other introduction, of the antigenic into the host, the host's system responds by producing large amounts of antibody to the antigen. Since the specific antigenic determinant of the manufactured antigen, i.e. the antigen formed of the synthetic peptide and the carrier, is the same as or at least immunologically equivalent to the determinant of the natural antigen of interest, the host becomes immune to the natural antigen. In the case where the invention is used as a vaccine, this is the desired result.

It is very often desirable to determine if a particular antigen is present as an aid, for example, in the diagnosis of a particular disease. Because the synthetic antigen is mono-specific to the single specific antigenic determinant of interest, antibodies to the antigen are also mono-specific to the antigen of interest. Perfect mono-specificity may not always be accomplished but cross-referencing to other antigenic portions of the antigen is avoided because only one immune response is possible by the antibody. Antibodies are harvested and prepared in any conventional procedure for use in diagnostic tests. It is common, for example, to label the antibody for identification and quantitative determination. Radiolabelling by the method of Greenwood, for example, (see Hunter, *Br, Med. Bull.* 30:18 (1974))) and fluorescent dye labelling are commonly used in immunoassays.

IV. Exemplary procedure — antigenic determinant unknown
Summary:

The accepted genetic structure of replication-competent murine leukemia viruses, such as Moloney, Rauscher, Friend and AKV, includes three genes. These genes, *gag, pol* and *env,* are arranged, in respective order, along the single-stranded RNA genome. They are transcribed from the integrated double-standard DNA provirus. The protein products of these three genes are understood in considerable detail. The *gag* gene encodes a polyprotein of about 65 kilodaltons which is proteolytically processed to proteins, amino to carboxy terminal, of 15, 12 30 and 10 kilodaltons, respectively. These proteins are found in the core of the virion, and one, p30, has been associated with virus tropism. The second gene, *pol,* is expressed as an apparent extension of the *gag* gene such that an approximately 180 kilodalton polyprotein containing both *gag* and *pol* components is observed. The polyprotein is processed to a 70 kilodalton protein which is the reverse transcriptase. This enzyme is responsible for copying the single-stranded RNA genome of an infecting virus into the double-stranded DNA structure which can integrate into host DNA. The third gene, *env,* encodes a polyprotein which is glycosylated and processed into components gp70 and p15E. Gp70 is the major envelope component and determines viral host range. It is sometimes found disulfide linked to p15E, a hydrophobic protein which may anchor gp70 to viral and cellular membranes. Messenger RNA molecules have been described which can account for the expression of these three genes.

We began our study at the 3' end of Moloney Murine Leukemia Virus (Mo-MuLV) because of our particular interest in the *env* gene which was thought to be the most 3' proximal gene. Here we discovered a new genetic coding region which lies on the 3' side of *env.* We provisionally name this the R-region because it is the coding region that is the furthest to the right in the genome. We have also chemically synthesized part of the R protein, raised antibodies to the synthetic peptide, and detected immunologically cross reactive material in infected cells.

The DNA sequence

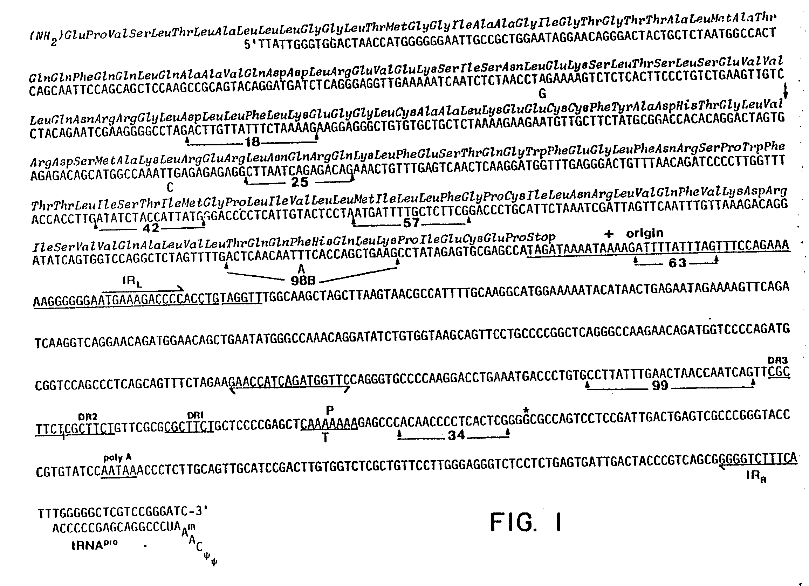

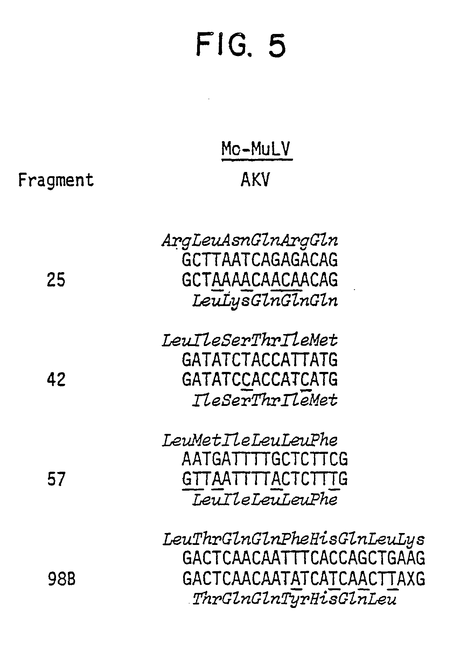

Figure 1 depicts the nucleotide sequence of the 3' end of the Mo-Mu-LV provirus. The 1123 nucleotide plus-strand sequence from the 3' LTR and carboxy terminal virus coding region was solved from an 1108 base pair long cDNA clone and extended slightly by sequence from a non-infectious clone. The protein sequence translated from the DNA appears above the nucleotide sequence. The first 34 amino terminal residues were determined by Copeland and Oroszlan and positions 10—34 match our sequence. The numbered oligonucleotides (25, 42, 57 and 98B) represent those which correspond to AKV virus and are shown in detail in Figure 5. The underlined region downstream from the R coding region is the origin of plus strand DNA synthesis. The regions marked $IR_L$ and $IR_R$ are the inverted terminal repeats which flank the LTRs. Within the LTR we recognize a 17 base long palindrome (underlined) and 3 direct repeats (DR 1, 2 and 3) which lie just upstream from the promotor Hogness-box (p) for the 5' end of the viral transcript. Further down the molecule is the poly A addition signal for the 3' end of the transcript.

We have also sequenced this region of a full-length virus clone that can be demonstrated by transfection studies to carry biologically-active Mo-MuLV coding sequences. This new DNA sequence agrees with the original except for the inconsequential differences described. We consider these differences to represent either variability in the population of biologically active genomes or artifacts due to the known sligbht unfaithfulness of the reverse transcriptase reaction which generated the clone we initially examined.

For orientation purposes, we indicate several features of this sequence that are important to the virus life cycle. Proceeding from 5' to 3' along the DNA strand corresponding to genomic RNA we see the coding region for most of p15E (the carboxy terminal *env* coding region); the origin of second- (positive) strand DNA replication; the inverted repeats, $IR_L$ and $IR_R$, which flank the portion of viral sequences that are duplicated at the 5' and 3' ends of the integrated provirus (the so-called LTRs) and make the DNA of the virus a transposon; and the poly A addition signal and presumptive 3' ultimate nucleotides preceding the poly A tail. Also indicated are features which are active at the 5' copy of the LTR, namely, the promotor for genomic expression and the first transcribed base, as well as a sequence slightly upstream from the promotor Hogness box containing 3 direct repeats of a 7-base long sequence. These repeats may (by positional argument) be involved in the control of transcription. We also notice within the 515 baselong terminal repeat a sequence of 17 bases which can form a self-contained inverted repeat (palindrome) but is of unknown (if any) function.

### A new protein predicted by DNA sequence

The new genetic region that we find can best be understood relative to what was previously thought to be the right-most gene of the provirus — the coding region for the viral protein p15E. The amino terminus of p15E can be positioned precisely by overlap between the protein sequence predicted by our DNA sequence and the protein sequence obtained by others. Three factors suggest that the carboxy terminus of p15E is defined as position 103: Copeland and Oroszlan determined the two C-terminal amino acid residues of p15E to be leu-val. We find such a couplet 103 positions from the p15E amino terminus. The predicted amino acid composition of p15E that one gets by assigning the carboxy terminus to valine at position is in excellent agreement with the observed composition. Finally, the apparent molecular size of p15E as determined on SDS gels is about 15 kilodaltons, a size estimate compatible with the mobility of a hydrophobic protein of 103 residues.

Surprisingly, we do not find a translational terminal codon in position 104. Instead, the open translational reading frame extends for 92 more triplets before encountering a stop. We conclude that the primary *env* gene protein product in fact contains three peptides; gp70, p15E and this newly identified protein, R. Therefore, we looked for the R protein.

### Chemical synthesis and detection of the R-protein in infected cells

We synthesized, by solid state methods several peptides of the predicted Mo-MuLV R-protein and raised antibodies to these synthetic peptides. Specifically, the C-terminal 15 residues (LTQQFHQLKPIECEP) were attached to the carrier molecule KLH and injected into 6 rabbits and 4 mice. We assayed the sera for the ability to immunologically precipitate a 36 residue synthetic substrate containing the 35 C-terminal residues of the R-protein preceded by [125]-tyrosine ([125]-YILNRLVQFVKDRISVVQALVLTQQFHQLKLPIECEP). Sera from all of the immunized rabbits and mice showed a positive response of 10 to 70-fold over normal serum, as shown in Table 1.

TABLE 1

| Animal # | Immunization schedule | Counts (0.1 min) $^{125}$I-36 amino acid poly peptide precipitated |
|---|---|---|
| normal rabbit serum | — | 432 |
| rb# 02809 | A | 4,867 |
| rb# 02810 | A | 15,359 |
| rb# 02623 | B | 13,243 |
| rb# 02623 later bleed | | 21,200 |
| rb# 02624 | B | 16,434 |
| rb# 02624 later bleed | | 18,436 |
| rb# 02625 | C | 5,548 |
| rb# 02625 later bleed | | 4,030 |
| rb# 02626 | C | 9,121 |
| rb# 02626 later bleed | | 19,799 |
| rb# 02618 | D | 559 |
| rb# 02619 | D | 566 |

TABLE 1 (Cont.)

Schedule A:  day 1—1 mg/kg in Freunds complete adjuvant subcutaneously in 4 foot pads and along back

CT15—KLH

day 7—repeat injections—Freunds complete adjuvant
day 14—repeat injections—Freunds complete adjuvant
day 21—bleed

Schedule B:  day 1—200 µg/rabbit in Freunds complete adjuvant subcutaneously (rabbits weighed about 2.5—3 kg so this was considerable reduction in dose)

CT15—KLH

day 7—50 µg/rabbit in Freunds incomplete adjuvant subcutaneously
day 14—50 µg/rabbit in alum (4 mg/rabbit)
bleed day 21 and 28
boost with CT15—KLH in alum, 50 µg/rabbit and bleed 7 days after boost

Schedule C:  Same as Schedule B except that initial dose was reduced in 50 µg/rabbit subsequent doses were also 50 µg/rabbit

CT15—KLH
Schedule D:  Same as Schedule C except with CT15-biotin-avidin

We then looked for the R-protein in lysates of MuLV producing SCRF 60A cells that had been labelled by a two hour $^{35}$S-methionine pulse (the R protein contains no tyrosine and so was not expected to label with iodine).

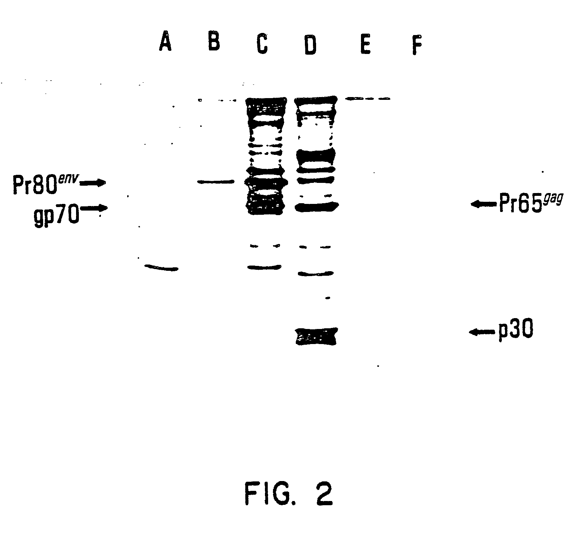

Figure 2 depicts the reaction of log phase SCRF 60A cells, which produce a virus indistinguishable from Mo-MuLV (16), were grown in Eagle's MEM with 10% fetal calf serum. The cells were labelled at 37°C at $2 \times 10^6$ cells/ml for 2 hours with $^{35}$S-methionine (940 ci/mmole, 100 µCi/ml) in Hank's Balanced Salts with 10% Eagle's MEM. Cells were chilled, washed with 0.15M NaCl, 10mM Sodium Phosphate (pH 7.5), extracted with 0.15M NaCl, 10mM Sodium Phosphate (pHH 7.5), 1% NP40, 0.5% Sodium Deoxycholate, 0.1% SDS, 2% Trasylol for 20 minutes at 0°C, then sonicated and spun at 12,000 × g for 5 minutes. The lysate was pre-cleared by reacting with 20 microliter of normal rabbit serum and 20 microliter of normal goat serum for 30 minutes at 0°C, followed by the addition of formalin fixed *Staph A* for 30 minutes and centrifugation for 15 minutes at 12,000 × g. Portions of the lysate were reacted with 5 µl of A) normal rabbit, B) rabbit anti-synthetic R pentadecapeptide, C) goat anti-gp70, D) goat anti-p30, or E) normal coat sera or F) anti-gp70 hybridoma (no. R1-16G07, ref. 17) culture media for 1 hour at 0°C. Complexes were collected with *Staph A* and after 2 washes with 500mM LiCl, 100mM Tris (pH 8.5) dissolved in loading buffer cleared of *Staph A*, and loaded on a 11% SDS-polyacrylamide gel. The gel was soaked in ENHANCE (NEN) for 90 minute, $H_2O$ for 60 minutes, dried and exposed to film. The bands indicated in the figure have been identified previously in this laboratory and by others.

Anti-R sera were prepared as follows. The carboxyterminal R pentadecapeptide (LTQQFHQLKPIECEP) was conjugated to keyhole limpet hemocyanin (KLH) through the cysteine sulphydryl. 63 µl of 15 mg/ml m-malimidobenzoyl-N-hydroxysuccinimide ester in N,N'-dimethylformamide was added dropwise with stirring to KLH (10 mg/ml) in 10 mM potassium phosphate (pH 7.0). After stirring for 30 minutes, the mixture was filtered and applied to a Sephadex G-25 column (0.1M phosphate, pH 6.0). The activated protein (2.3 ml) was mixed with 0.1 ml of the R pentadecapeptide (10 mg/ml in 0.1M potassium phosphate, pH 7.3, 5 mM EDTA) and the solution adjusted to pH 6.5, stirred for 4 hours at room temperature and chromatographed on Sephadex G-100 (0.1M ammonium bicarbonate pH 9.5). The conjugated protein was collected and used directly for immunization.

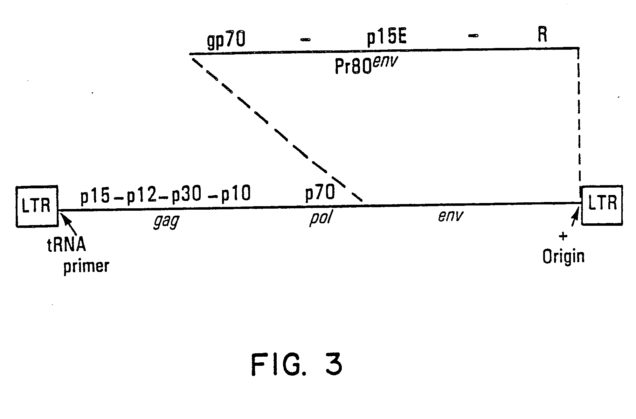

As can be seen in Figure 2, an anti-R serum detects a protein with an apparent molecular size of approximately 80 kilodaltons in infected cells. All of the individual immune rabbit and mouse sera detected proteins with the same molecular size. Because the nucleotide sequences showed that p15E and R were in the same reading frame, we expected that this molecule was, in fact, the *env* precursor. Antibodies to gp70, including an anti-gp70 hybridoma, detect a molecule with the same apparent molecular size as that precipitated by anti-R, confirming that we are observing the *env* precursor. To rule out the possibility that those were two distinct molecules with a fortuitous correspondence in molecular size, we did two experiments. First, when the lysates are pre-cleared with anti-R sera, the 80 kilodalton radioactive target for anti-gp70 sera is removed. Conversely, clearing the lysate with anti-gp70 sera removes the R determinants. Secondly, proteolytic peptide fingerprintng of the two 80 kilodalton molecules precipitated by anti-R and anti-gp70 shows them to be identical. Thus we have identified the 80 kilodalton band as the complete *env* gene polyprotein product containing gp70, p15E (as shown by others), and R determinants, thereby proving the structure suggested in Figure 3.

Figure 3 depicts a revised genetic map for Mo-MuLV provirus. The 515 nucleotide-long LTRs flank the protein coding region of the virus. The origins of minus strand (tRNA primer binding site) and plus strand DNA replication are just into the body of the virus from the LTRs at each end. The primary *gag* gene product (Pr65$^{gag}$) is processed to components, N to C, p15, p12, p30 and p10. The final product of the *pol* gene is 70 kilodaltons. The *env* polyprotein, called Pr80$^{env}$, consists of components, N to C, gp70, p15E and the newly identified R protein.

The first amino acid of R, namely that which immediately follows the valine carboxy terminus of p15E, is an arginine. Since basic residues are often found at proteolytic clip sites, it is possible that R is proteolytically processed from the *env* polyprotein at this site. Using different gel electrophoresis conditions, we were able to detect a protein of estimated molecular size 12 kilodaltons specifically precipitated by anti-R serum.

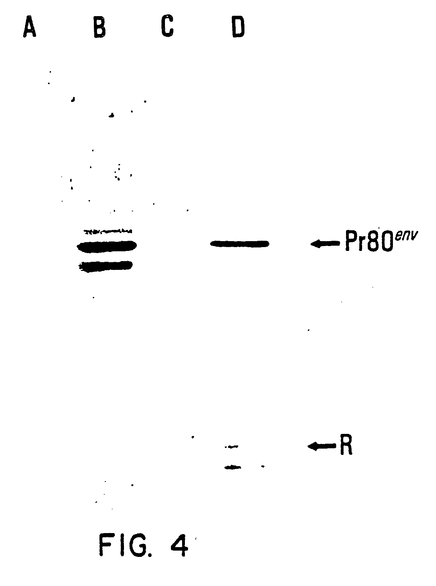

Figure 4 depicts the detection of the R protein. An $^{35}$S-methionine labelled lysate of SCRF 60A cells (as described in the Figure 2 legend) was divided into 4 equal portions and immunologically precipitated with A) normal goat, B) goat anti-gp70, C) normal rabbit, or D) rabbit anti-R serum, collected with *Staph A* and electrophoresed on a 5—17.5% SDS polyacrylamide gradient gel.

Also, we have observed, by indirect immunofluorescence, R protein determinants on the plasma membrane of SCRF 60A cells. It cannot be determined from our experiments at what time the p15E cleavage occurs and we are open to the possibility that the true gp70 membrane anchor is the 22 kilodalton protein containing p15E and R peptides. In that case, the proteolytic clip which generates p15E and R occurs during their isolation. We have not observed a 22 kilodalton protein with R determinants.

The R-gene is present in another virus. Pederson and Haseltine determined the nucleotide sequences of the large ribonuclease T1 fragments of AKV virus. (AKV virus is an endogenous leukemia virus of AKR mice.) We could identify by computer search several of these fragments as having partial homologies to skeins of our Mo-MuLV sequence. Our defined order makes a reasonable match with the order obtained experimentally by Pederson, Crowthere and Haseltine. The matches within the R-region are indicated in Figure 1 and are detailed in Figure 5.

Figure 5 illustrates a comparison of the R-gene in two viruses. The oligonucleotide sequences determined by Pederson and Haseltine for AKV were matched by computer to our Mo-MuLV sequence. Those matches within the R-region are shown, Mo-MuLV above AKV, with the corresponding translated amino acids. The numbers in the margin are those used by Pederson and Haseltine. Differences between the nucleotides are underlined. In fragment 98B, the phenylalanine codon in our cDNA sequence was a tyrosine codon in the infectious clone and is a tyrosine in AKV. The X is fragment 98B is an unsolved position for AKV.

We glean two things from these alignments. First, we notice that the matched fragments, although not chance pairings, are not wholly conserved. This, perhaps, warns us as to what to expect from futgure comparisons of sequences of related viruses. Second, we notice that matches within the R-region indicate that AKV probably encodes an R-protein much like that of Mo-MuLV. Specifically 1) none of the AKV differences introduces nucleotides which mandate a premature translational stop, 2) several (9) AKV/Mo-MuLV nucleotide differences are in third positions in triplets and do not alter the coded protein sequence, 3) some nucleotide differences do change R-protein amino acids, but the replacements tend to be conservative or synonymous (as indicated in Figure 5) such as phenylalanine to tyrosine in spot 98B, asn-gin-arg to lys-gin-gin in spot 25, or met to leu in spot 57. All these substitutions are rated as positive correlations by the Dayhoff mutation matrix. Additionally, a preliminary sequence obtained by Winship Herr through 217 nucleotides of the R-region of AKV shows an open reading frame containing 56 nucleotide differences with our Mo-MuLV sequence. These 56 differences make only 6 amino acid alternations, all of which are neutral. These observations indicate that the R-protein sequence is highly conserved in the two viruses.

## Comments about the R-protein

The entire amino acid sequence of the R protein does not closely correspond to any protein sequence in the Dayhoff Atlas. Some subregions of R, however are reminiscent of subregions of known proteins such as dehydrogenases and proteases, perhaps implicating domains of the R-protein in nucleotide binding or protein-protein interactions.

The R-protein might function simply as a structural viral protein. In this regard, the very hydrophobic domain between position 32 and 61 would suggest that R is inserted into cellular or viral membranes where it serves to anchor other viral proteins. As mentioned above, we already have evidence from florescence microscopy that R is present in the plasma membrane of infected cells. Alternatively, the R protein might be involved in other sorts of functions. As originally noted by Fisher, genes and their regions of action seem to cluster. The R-region is nestled snuggly between the p15E coding region and the origin of positive strand replication and LTR. It is, therefore, possible that R functions in replication or transposition (integration). On the other hand, the R-protein could play a role in viral transformation. A perplexing mystery of the leukemia viruses has been that, although they transform lymphoid tissues, they carry no apparent transforming genes.

As a transforming protein, R could act directly or could interact with another protein. In either case, two important points must be explained. Firstly, although they replicate in virtually all cell types of the mouse, these viruses only transform specific cells. Secondly, changes in the gp70 protein, which is encoded upstream from the R-protein, are highly correlated with leukemogenicity of various viruses notably those called MCF. Therefore, we imagine either that gp70 and R interact directly (perhaps through disulfide linkage) and have, in the Moloney case, a T-cell specific action or that gp70 triggers some cell-type specific event which renders the T-cell sensitive to the leukemogenic effect of the R protein. What would cause the virus to maintain this gene is unknown.

Our work, as described above, presents not only a new body of information about the system we selected to provide or to disprove the invention but provides a general method to access proteins by nucleotide sequences. In general, where the primary goal is simply to manufacture a synthetic vaccine to a natural antigen, not all of the studies, investigations and proofs of the example are necessary, as some of these investigations were aimed at obtaining information rather than production of vaccines or antibodies.

The steps taken in our first proof of the method include:

(a) If one is starting with an antigen of interest in which the antigenic determinant peptide has not been characterized, the first requirement is to identify and characterize this peptide. In the example, the existence of the determinant region was not even known but was proved. Also, in the example, since the virus was composed of RNA, it was necessary to copy to DNA by transcription.

(b) In order to get sufficient quantities for convenient handling, the antigen cDNA was inserted into a known plasmid and large amounts of the gene were grown. This step is not necessary, of course, if sufficient amounts of the DNA are available, or if the structure of the DNA or peptide is known, and was carried out simply for our working convenience.

(c) The nucleotide sequence of the cDNA was determined. This step would not be necessary if the peptide of interest had been characterized. Likewise, the step would not be necessary if one chose to characterize the protein directly rather than by means of its genome. The present study, which included the discovery that the protein had been cleaved or degraded and that there was an antigenic determinant site missing, establishes the very general applicability of the invention and opens the door to the manufacture of vaccines and diagnostic products for antigens which do not appear in the infecting organism at all.

(d) Once the nucleotide sequence is known, the genome determines the amino acid sequence of the protein and the peptide to be duplicated synthetically can be selected. Any area of the protein is a suitable starting point. The absence of a region of cross-reactivity with host cells indicates an advantageous site for synthesis to begin. Having made the selection, a peptide of the desired length is synthesized using, in the above example, the Merrifield et al method. The peptide should be at least 4 mer, and generally at least 8 mer. Longer peptide chains assure greater specificity but also require greater time in synthesis. Peptides of 8 to up to several hundred mer will generally be suitable. It will be noted that the immune response to the 15 mer antigenic determinant was specific both to a 35 mer (plus radiolabel) determinant and the natural antigen.

(e) The specific antigenic determinant which was synthetically manufactured, and was thus free of extraneous proteins, endotoxins, cell fragments, viral genomes, etc., is then conjugated with a suitable carrier to form the vaccine in which the specific antigenic determinant is the synthetic peptide.

(f) The vaccine, when injected into the host, immunized the host and initiated the production of antibodies.

(g) The antibodies proved oligospecific for larger synthetic antigenic determinant and to the natural antigen.

(h) The antibodies bound to and inactivated the virus.

Thus, the invention was successful in (i) predicting and synthesizing a heretofore unknown antigenic determinant, (ii) manufacturing a vaccine which initiated the desired immunoresponse and (iii) manufacturing an antibody which monospecifically bound the natural antigen and neutralized the virus.

Hepatitis and influenza vaccine

Summary:

Thirteen peptides corresponding to amino acid sequences predicted from the nucleotide sequence of the hepatitis B surface antigen (HB$_s$Ag) were chemically synthesized. The free or carrier-linked synthetic peptides were injected into rabbits and seven of the thirteen elicited an anti-peptide response. Antisera against four of the six soluble peptides longer than 10 amino acids were reactive with native HB$_s$Ag and specifically precipitated the 23,000 and 28,000 dalton forms of HB$_s$Ag from Dane particles. Since the hepatitis molecule was not chosen for study because of any stuctural feature which suggested unique opportunities for success, these results indicate that the strategy is general and should work for any molecule as long as enough domains are studied. Peptides such as these are useful as vaccines.

Two genes whose nucleotide sequences were known and whose protein products were of both theoretical and practical interest were selected. The first was the major envelope protein of the hepatitis B genome, a molecule which, because of its extreme hydrophobicity, offered an interesting challenge to the technology. Second, the humaglutinin of influenza virus was selected because its complete crystalographic structure is known (Wilson, I. A., Skehel, J. J. and Wiley, D. C. (1981) *Nature*, pp. 366—73), and thus one could correlate how antibodies to protein domains of known molecular location perturb virus infectivity and, in fact, what the structural correlates of antigenicity are for the molecule.

The hepatitis B surface antigen

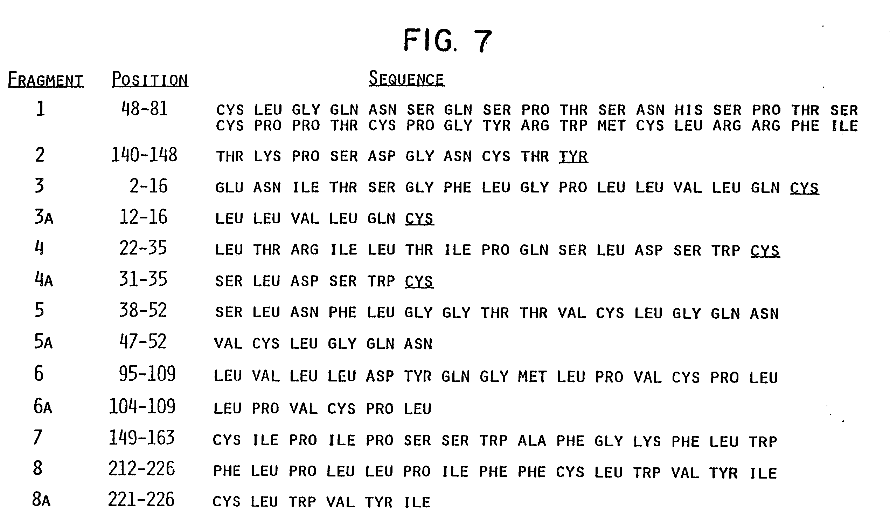

The hepatitis B virus surface antigen is a glycosylated protein and is the major surface antigen of 42 nanometer particles (Dane particle) of hepatitis B virus. The HB$_s$Ag contains group and type specific determinants and is thought to be the major target of neutralizing antibody. Purified preparations of HB$_s$Ag are physically heterogenous and consist of at least seven polypeptides ranging in molecular size from 23,000 to 97,000 daltons. By mass the major HB$_s$Ag component has a molecular size of 23,000 daltons. (Peterson, D. L., Roberts, I. M. and Vyas, G. N. (1977) *Proc. Nat. Acad. Sci.* U.S.A., 74, 1530—1534). Immunological studies showed that the proteins of different sizes share common determinants suggesting that the physical polymorphism reflects different degrees of glycosylation and aggregation. The amino acid sequence of the 226 amino acid long HB$_s$Ag deduced from the published nucleotide sequences is given in Figure 7. Overall, the HB$_s$Ag is an exceedingly hydrophobic molecule which is rich in proline and cysteine residues. The HB$_s$Ag was studied by the unpublished computer program of Kyte and Doolittle which makes a running average of local hydrophobicity and has been shown to be highly predictive of internal and external residues of proteins whose structures are known. If the HB$_s$Ag is considered in terms of domains, one can discern three hydrophobic and two "hydrophilic" areas in the molecule. For simplicity, reference is made to hydrophilic domains, but, in fact, the molecule is so hydrophobic that it is probably more accurate to think in terms of hydrophobic and not so hydrophobic domains. The largest and most hydrophobic region spans approximately positions 80—110. This hydrophobic domain is flanked by two "hydrophilic" domains encompassing positions 45—80 and 110—150. The other two hydrophobic domains are found at the N- and C-termini. Most of the cysteines are clustered in the two "hydrophilic" domains. Overall then, one has the picture of a hydrophobic molecule with potential for complex conformation dictated by frequent bends at prolines and intrachain disulphide bonds at cysteins. Such a structure is consistent with the known resistance of the molecule to denaturation and digestion by proteolytic enzymes. (Millman, I., Loeb, L. A., Bayer, M. and Blumberg, B. S. (197) *J. Exp. Med.* 131, 1190—1199). Thus, one might have expected that most of this molecule's antigenic determinants would be formed by amino acids distance in the linear protein sequence but held close together in space by the molecule's tertiary structure. As such, HB$_s$Ag is an excellent test case for generalizing the use of continuous amino acid sequences in designing synthetic antigens according to the present invention.

Materials and methods

Synthesis of peptides

For this study, peptides were synthesized using the solid-phase methods developed by Merrifield and his colleagues. Each synthetic peptide was subjected to acid hydrolysis in vacuo (6N, HCl, 110°C, 72 hours) and the amino acid composition determined. No attempt was made to remove multimeric forms since the sole use of the peptides was as immunogens.

Coupling of synthetic peptides to carrier protein

All peptides except 1, 3a, and 7 were coupled to the carrier protein KLH (keyhole limpet hemocyanin) through the cysteine of the peptide using MBS (m-maleimidobenzoyl-N-hydroxysuccinimide ester) as the coupling reagent. In general, 5 mg peptide, dissolved in PBS (pH 7.5) or Na-Borate buffer (pH 9.0) was coupled to 3—4 mg KLH-MBS. The pH for dissolving the peptide was chosen to optimize peptide solubility and content of free cysteine determined by Ellman's method (Ellman, G. L. (1959), *Arch, Biochem, Biophys,* 82, 70—93). For each peptide, 5 mg KLH in 0.25 ml PBS was reacted with MBS dissolved in dimethyl formamide at a molar ratio of KLH:MBS of 1:40 and stirred for 30' at room temperature. KLH-MBS was then passed through Sephadex G-25 and washed with PBS to remove free MBS. KLH recovery from the peak fractions of the column eluate, monitored by O.D. 280, the estimated to the about 80%. KLH-MBS was then reacted with 5 mg peptide, adjusted to pH 7—7.5 and stirred for 3 hours at room temperature. Coupling efficiency was monitored with radioactive peptide. In general, 25—50 molecules of peptide were coupled per 100,000 daltons of KLH.

Preparation of anti-peptide antibodies

Rabbits were immunized according to the following schedule. 1) 200 µg peptide-coupled KLH in complete Freund's adjuvant (1:1) subcutaneously on day 0,2) 200 µg in incomplete Freund's adjuvant (1:1) subcutaneously on day 14, 3) 200 µg with 4 mg alum intraperitoneally on day 21. Animals were bled 4 weeks and 15 weeks after the first injection. Peptide 1 was injected without KLH (1 mg/injection) according to the same schedule.

Immune precipitation of synthetic peptides

The reactivity of the various anti-peptide sera was determined by their ability to immunologically precipitate radioiodinated target proteins. Peptides were labelled with [125]I by the chloramine T reaction if they contained tyrosine, or with Bolton-Hunter reagent. Highly purified envelope preparations (a gift from J. Gerin) were labelled by chloramine T. Radioiodinated targets were either suspended in PBS or in RIPA (0.15M NaCl, 10 mM sodium phosphate pH 7.5, 1% NP40, 0.5% sodium deoxycholate, 0.1% SDS) and reacted ($5 \times 10^6$ cpm/reaction) at 0°C with 5 µl of test serum or normal rabbit serum for 1 hour and precipitates collected with *Staphylococcus aureus*. Pellets were washed with RIPA, then twice with 500 mM LiCl, 100 mM tris (pH 8.5) and counted. Variability was about 20% in duplicate determinations.

Polyacrylamide gel electrophoresis of immune precipitates

Purified Dane particles (a gift of W. Robinson) were suspended in RIPA and radioiodinated with chloramine T. The preparation was precleared twice by incubation at 0°C with normal rabbit serum for 30 minutes, and with formalin-fixed *S. aureus* for 30 minutes followed by centrifugation (5 min. at 12,000 g) and then incubated with 5 µl of normal, anti-peptide 3 or anti-peptide 4 serum. Precipitates were collected and washed as above, suspended in gel loading buffer, boiled, centrifuged to remove *S. aureus*, and electrophoresed on a 5—17% acrylamide SDS gel, and autoradiographed.

Results
Selection of peptides for synthesis

Considerations concerning the physical structure of the HB$_s$Ag as well as variations amongst the three published nucleotide sequences dictated which peptides were selected for chemical synthesis. In general, we tried to select regions so as to span as large a portion of the protein sequence as possible with peptides containing a cysteine residue to allow coupling to a carrier protein. If the nucleotide sequence did not predict a cysteine in a region of interest, one was added to the C-terminus for purposes of coupling. The peptides synthesized are underlined in Figure 1 and listed in Figure 8.

The entire molecule was not synthesized because some regions were judged less likely to succeed than others. The area between 81—94 was avoided because of its extreme hydrophobicity. Synthetic peptides corresponding to this sequence would not be expected to be soluble, and even if an antibody to them could be raised, one might not expect that this region would be located on the surface of the native molecule. For similar reasons, a large portion (positions 164—211) of the hydrophobic C-terminal domain was not studied. The region between positions 110—140 was avoided because there was not a consensus in this region among the three published nucleotide sequences. (Valenzuela, P., Gray, P., Quiroga, M., Zaldivar, J., Goodman, H. M. and Rutter, W. J. (1979), *Nature* 280, 815—819. Pasek, M., Goto, T., Gilbert, W. Zink, B., Schaller, H., Mackay, P., Leadbetter, G. and Murray, K. (1979); *Nature* 282, 575—579. Galibert, F., Mandart, E., Fitoussi, F., Tiollais, P. and Charrey, P. (1979), *Nature* 281, 646—650). Peptides corresponding to the extreme N and C-termini of the molecule were included because of previous success in using these regions of molecules where the complete tertiary structure was unknown. (Sutcliffe, J. G., Shinnick, T. M., Green, N., Liu, F-T, Niman, H. L. and Lerner, Ra. A. (1980), *Nature* 287, 801—805. Walter G., Scheidtmann, K-H, Carbone, A., Laudano, A. and Doolittle, R. F. (1980), *Proc. Natl. Acad. Sci.* U.S.A. 5179—5200). Remaining peptides were selected to correspond to hydrophilic domains of the molecule, as well as to proline- containing junctions between hydrophilic and hydrophobic domains where the molecule might be expected to turn and expose "corners". Peptides 3a and 7 were found to be insoluble and hence, were not pursued.

Antibodies to some synthetic peptides react with native $HB_sAg$

Before testing reactivity to native $HB_sAg$, it was important to ensure that an antibody response to the synthetic peptide had occurred. As seen from the data in Table 2, when coupled to KLH, six of the ten peptides were immunogenic as judged by the ability of the antisera to precipitate the radioiodinated peptides.

TABLE 2
Reactivity of anti-peptide sera against peptide and viral envelope

| | | Antibody titer[+] VS: | | | |
|---|---|---|---|---|---|
| | | Peptide | | Viral envelope | |
| Peptide | Rabbit | 4 Weeks | 15 Weeks | 4 Weeks | 15 Weeks |
| 1* | 03288 | 6.4 | 8.4 | 8.3 | 13.4 |
| 1* | 03289 | 8.6 | 7.6 | 28.0 | 52 |
| 1** | 03300 | 3.7 | — | 1.0 | — |
| 2 | 03370 | 2.1 | 1.3 | 2.4 | 1.0 |
| 2 | 03371 | 2.7 | 1.7 | 1.1 | 0.9 |
| 3 | 03302 | 1.6 | 20 | 2.0 | 5.8 |
| 3 | 03303 | 5.2 | 15.8 | 14.0 | 36 |
| 3a | | NT(insoluble) | | | |
| 4 | 03220 | 7.9 | 7.5 | 32.5 | 92 |
| 4 | 03221 | 4.8 | 6.1 | 7.2 | 71 |
| 4a | 03211 | 1.0 | — | 1.0 | — |
| 4a | 03213 | 1.0 | — | 1.0 | — |
| 5 | 03308 | 8.5 | — | 1.0 | — |
| 5 | 03310 | 5.9 | — | 1.0 | — |
| 5a | 03305 | 5.3 | — | 1.0 | — |
| 5a | 03307 | 5.8 | — | 1.0 | — |
| 6 | 03306 | 51.0 | 85 | 75.6 | 113 |
| 6 | 03309 | 17.7 | 83 | 9.5 | 37 |
| 6a | 03169 | 12.3 | — | 1.0 | — |
| 6a | 03212 | 11.0 | 25 | 1.0 | 1.0 |
| 7 | | NT (insoluble) | | | |
| 8 | 03219 | 1.0 | — | 1.0 | — |
| 8 | 03210 | 1.0 | — | 1.0 | — |
| 8a | 03215 | 1.0 | — | 1.0 | — |
| 8a | 03216 | 1.0 | — | 1.0 | — |

[+]Antibody titer is expressed as counts precipitated by test serum divided by counted precipitated by normal serum.
*Injected at pH 5.3
**Injected at pH 8.5

Only peptides 4a, 8, and 8a failed to elicit an antipeptide response. Peptide 2 elicited only a marginal response. Peptide 1 was an effective immunogen without requiring coupling to KLH. Although the extent was graded with time, early bleeds indicated the direction of the responses.

To determine whether the antibodies raised against the various peptides could react with the HB$_s$Ag molecule, we assayed their ability to immunoprecipitate, radioiodinated HB$_s$Ag that had been purified from hepatitis B Dane particles. When the HB$_s$Ag was suspended in RIPA buffer, four of the seven antibodies that reacted against the appropriate peptide also precipitated HB$_s$Ag. Specifically, antibodies to peptides 1, 3, 4 and 6 reacted with purified HB$_s$Ag, whereas antibodies to peptides 5, 5a and 6a failed to react, as did those antisera which did not see their target peptide (4a, 8, and 8a). Again, peptide 2 antisera gave a marginal reactivity. In the study presented in Table 2, it is clear that there is variability between sera of rabbits which received identical treatments. In all but one animal, no. 03302, the early bleeds were predictive of the 3 month response. Peptide no. 8 was not very soluble and thus the failure of two rabbits to respond to it must be considered tentative in light of our inability, because of solubility, to determine how efficiently it coupled to KLH.

Several interesting features concerning individual peptides as immunogens are evident in Table 2. Peptide number 6 is highly immunogenic and induces antibody reactive with itself as well as the native HB$_s$Ag. However, peptide no. 6a (the C-terminal 6 amino acids of peptide 6) although capable of inducing antibody to itself does not induce antibody reactive with native HB$_s$Ag. On the other hand, no. 4 is capable of inducing antibody to itself and native HB$_s$Ag, whereas the C-terminal hexamer (peptide no. 4a) does neither. Peptide no. 1 is of special interest from two points of view. First, its immunogenicity does not depend on a carrier, perhaps because it is of sufficient length to induce antibody by itself. But, more interesting is the fact that its ability to induce antibody reactive with native HB$_s$Ag depends on the pH used to solubilize the immunogen. At pH 5.3, peptide no. 1 is completely soluble and expresses 62% free cysteine. Antibodies raised against the peptide solubilized at this pH recognize the target peptide as well as the native HB$_s$Ag. In contrast, at pH 8.5, the peptide is barely soluble (less than 15%) and expresses no free cysteine. When injected at this pH, the peptide elicits a poor response to itself and none to HB$_s$Ag.

Although RIPA buffer would not be expected to denature HB$_s$Ag, we wished to study the immune reactivity of the molecule under more physiological conditions. Accordingly, the antigen was suspended in PBS (0.15 m NaCl 10 mM sodium Phosphate pH 7.5) and reacted with various anti-peptide sera. All sera reacted with the HB$_s$Ag in PBS with about the same efficiency as in RIPA (data not shown). Thus, the antibodies recognize the molecule under conditions which approximate its native condition. Therefore, antibodies against such peptides might be expected to function *in vivo* as well as *in vitro*.

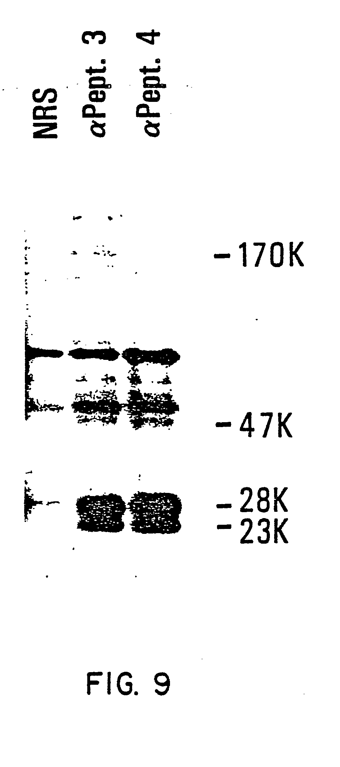

To determine which molecule(s) of Dane particles were reactive with antibodies to these synthetic peptides, purified Dane particles (serotype adw) were disrupted with detergent and the proteins radioiodinated. The labelled proteins were precipitated with the various antipeptide sera and the components present in the precipitates were analyzed on SDS-polyacrylamide gels. Two major components with approximate molecular sizes of 28,000 and 23,000 daltons were specifically precipitated from Dane particles by antibodies reactive with native HB$_s$Ag, Figure 9. The 28,000 and 23,000 dalton species correspond to the previous described (Dane, D. S., Cameron, C. H. and Briggs, M. (1970) Lancet 695—698. Peterson, D. L., Roberts, I. M. and Vyas, G. N. (1977) *Proc. Natl. Acad. Sci.* U.S.A. 74, 1530—1534. Shih, J., and Gerin, J. D. (1977) *J. Virol.* 21, 347—357) two major forms of HB$_s$Ag (I and II) which differ in their degree of glycosylation. In addition, antisera against peptide 3 (Figure 3) and peptide 6 (data not shown) also reacted with proceins of about 47,000 and 170,000 dalton mobilities, which presumably represent multimeric forms or precursor molecules. The 47,000 dalton species is most likely the dimeric form of HB$_s$Ag (Mishiro, S., Imai, M., Takahashi, K., Machida, A. Gotanda, T., Miyakawa, Y. and Mayumi, M., (1980), *J. Virol.* 124, 1589—1593. Koistinen, V., (1980), *J. Virol.* 35, 20—23. Thus, the antibodies are directed against a protein found in the known etiological agent of hepatitis B.

The concept of molecules which are precursors to HB$_s$Ag is consistent with the data of Robinson in which tryptic fingerprints shown that some spots of the higher molecular weight forms correspond to those of HB$_s$Ag whereas other spots do not (Robinson, W., personal communication). Whereas all antibodies reactive with HB$_s$Ag see the 23,000 and 28,000 dalton forms of HB$_s$Ag in Dane particles, only some see the higher molecular weight forms. Presumably, the conformation and/or the degree of glycosylation of the larger forms in such that the peptide in question is hidden. Alternatively, the processing of the precursor may include binding to proteins or cellular structures which hide the target peptide.

Following development of the foregoing, the following peptide was determined, by the same methods, to have specific antigenic determinant characteristics for Hepatitis B:

PheProGlySerSerThrThrSerThrGlyProCysArg
ThrCysMetThrThrAlaGlnGlyThrSerMetTyrProSerCys

The left half was also a specific antigenic determinant, and, as discussed separately, regions of six or more amino acid peptides selected from known antigenic determinants also function as specific antigenic determinants when prepared and proved by the method described herein.

Influenza

The general procedure was applied in the production starting from the genome of influenza strain X-47 (M. Verhoeyen, R. Fong. W. Min Jou, R. Devos, D. Heijlebroek, E. Samon & W. Fiers, (1980), *Nature* 286, 771;

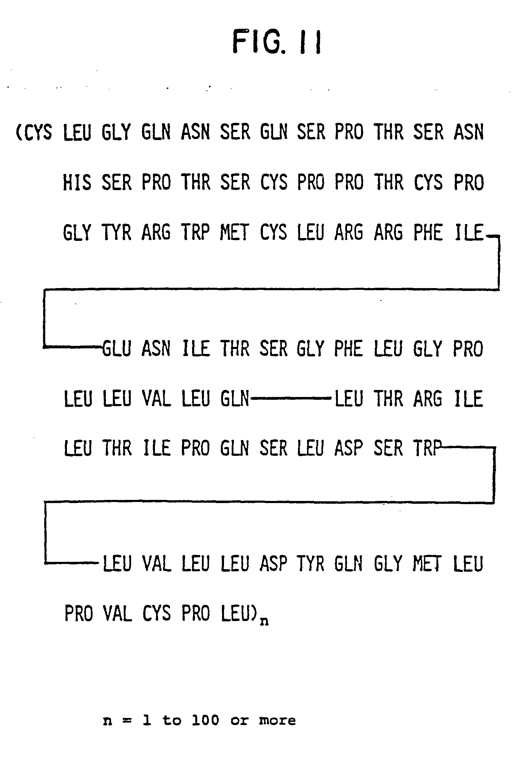

A. G. Porter, C. Barber, N. H. Carey, R. A. Hallewell, G. Threlfall, J. S. Emtage, (1979) *Nature 282*, 471) and using information from related species to predict potential specific antigenic determinant regions (I. A. Wilson, J. J. Skehel, D. C. Wiley (1981) *Nature 289,* 366). A number of the peptide regions coded from the genome regions marked in Figure 11 have been synthesized and the general method has been proved. Additional proof is being developed and there is no doubt that the method is general in providing an approach to the manufacture of antigens and antigenic and antibody preparations of the type described herein.

Integral antigen carriers

In general, it is necessary to attach the specific antigenic determinant peptide to a carrier to initiate an antibody response in a host. In some instances, if the peptide is large enough, the peptide may serve as the carrier. While many carriers are available for laboratory usage, the number of carriers which are approved for use in clinical preparations is very limited indeed. These problems are obviated by one facet of the present invention: the preparation of poly(peptide) polymers and copolymers.

Having identified the specific antigenic peptides and proved that they produce antibodies to the organism of interest, when one may prepare an antigen which consists essentially of specific antigenic determinant peptides linked together as homopolymers of repeating units of the same peptide, as copolymers of two or more specific antigenic determinant peptides (which may initiate antibodies to the same or a different naturally occurring organisms) or as copolymers with other peptides, some or all of which may not be antigenic determinants. The techniques of linking peptide chains together is well known; however, until now it has been neither possible to accomplish the resulting product described, which is a new result, nor proposed to prepare poly(peptide specific antigenic determinant) polymers or copolymers.

Exemplary of such an antigen is the poly(specific antigenic determinant peptide) of Hepatitis B shown in Figure 12.

Liposomes

Liposomes which comprise specific antigenic determinant surface moieties are prepared by linking a plurality of the peptide specific antigenic determinants to a fatty acid moiety, e.g. a stearoyl moiety, and reacting the resulting stearoyl peptides with a lipid rich nucleus. The stearyl moieties orient themselves toward and "dissolve" into the nucleus resulting in a liposome which displays a plurality of specific antigenic determinant peptides. All peptides may be the same specific antigenic determinant, or a number of specific antigenic determinants for the same or different naturally occurring antigens for the same or different organisms may be attached as part of the specific antigenic determinant liposome. Antigenic liposomes of the type referred to have not contemplated or possible heretofore.

Discussion

In broad outline the present studies illustrate that one can take a given nucleotide sequence, chemically synthesize several peptides from various domains of the predicted protein and, with some of these, raise antibodies reactive with the native structure. Since the hepatitis molecule was not chosen for study because of any structural feature which suggested unique opportunities for success, our results suggest that the strategy is general and should work for any molecule as long as enough domains are studied. As for the "rules" we have learned to date, peptides or limited solubility or those containing fewer than 6 amino acids are a poor choice. All the productive peptides contained one or more prolines, a fact consistent with its known presence in turns. In this study, 4 or 6 soluble peptides, ranging from 10—34 residues, proved useful. General application of this technique to finding unknown proteins from the known nucleotide sequences of their gene is now feasible.

Previous studies on the hepatitis B surface antigen concluded that it was a molecule critically dependent on conformation for preparation of antibodies reactive with the native structure. Vyas and colleagues suggested that reduction and alkylation of the disulfide bonds of the hepatitis B antigen resulted in complete loss of antigenicity (Vyas, G. N., Rao, K. R. and Ibrahim, A. B. (1972) *Science 178*; 1300—1301). By contrast, the present results show that there are determinants in the $HB_sAg$ which are not dependent on any conformation other than that which can be attained by short peptides. When the two studies are considered together one concludes that a linear sequence as a part of a larger denatured structure, albeit alkylated, will not elicit antibodies reactive with the native molecule whereas that same sequence free from constraints of neighboring amino acids will elicit such antibodies.

There are domains of the $HB_sAg$ which remain to be explored using synthetic polypeptides. We know little about the molecule between positions 110—140 and 162—210. The hydrophilic region between 110 and 140 is of particular interest because of the high degree of variation among the various different sequences. Interestingly, in the study by Pasek et al, the plasma used as a source of Dane particles was of complex serotype (adw and agw) and these authors noted micro-heterogeneity in the region of sequence corresponding to $HB_sAg$. Perhaps the sequence variation between 110—135 corresponds to the domain of the molecule conferring type specificity to the $HB_sAg$. The region spanning position 40—50 also shows significant heterogeneity among the 3 nucleotide sequences. In this regard, it is of interest that antibodies made against peptide no. 5, which corresponds to the region predicted from the nucleotide sequence of Pasek et al, do not react without test envelope (serotype adw). This may be due to the fact that in this region, perhaps a second region of type specific variation, the peptides we chose did not correspond to that of the envelope we used.

The results presented here establish the generality of one's ability to synthesize peptides predicted from nucleic acid sequences and raise antibodies reactive with the native molecule. Such antibodies are unique reagents insofar as they react with a small region of the native molecule which is known in advance to the experimentor. Thus, antibodies made in this way differ from hybridomas which, although useful at the outset for studies of whole molecules, must be further characterized for fine structure analysis of protein domains.

Synthetic peptides prepared using nucleotide sequences as blueprints should prove to be ideal for use in vaccination. For example, a combination of polypeptides (such as 1, 3, 4, 6 of this study) might provide broad protection against hepatitis B virus, thereby obviating biological variables such as serotypic diversity and antigenic drift of the infectious agent as well as the individuality of the host immune response.

It is to be clearly understood that substitutions of individual amino acids may be made to result in equivalent specific antigenic determinants, as is known, and antigenic determinants fully equivalent, when prepared and proved as provided herein, may differ in a limited number of amino acids in the peptide sequence. All this is contemplated within the scope of this invention.

General application

The general approach of this invention is applicable to all pathogens in which there is an associated oligopeptide antigenic determinant which initiates an immunological response in the host.

Example—Bacteria

For example, where the pathogen is bacterial, and introduction of the bacteria into the host initiates production in the host of antibodies which tend to bind and neutralize the bacteria, the portion of the bacterial genome which encodes a probable antigenic determinant is identified using known genetic techniques. The gene of interest is then cloned and its nucleotide sequence is determined using known DNA mapping techniques. From the nucleotide sequence and the genetic code the amino acid sequence of the probable antigen protein region is determined. A large number of potential antigenic determinants are then chemically synthesized, coupled to a carrier to form a potential antigen and introduced into a suitable host. The potential antigen thus formed which initiates production of antibodies which bind and neutralize the bacteria, and are not deleterious to the host, is determined by known immunochemical techniques. The antigen thus determined to induce the desired immunological protection is then manufactured in any desired scale and may be used to produce protective antibodies in a host. Antibodies may be harvested to prepare oligospecific antibody, for use in diagnosis for example. This is, of course, exactly the procedure described initially for the preparation of an anti-viral vaccine, except that the point of focus is bacteria rather than virus.

Example—Diseases

The method of the invention may be used, for example, in the manufacture of vaccines to prevent and/ or antibody diagnostic preparations to identify bacterial diseases in animals such as anthrax (Bacillus anthracis), blackleg (Clostridium chauvoei), and hog cholera (Erysipelothrix rhusiopathiae), as well as virus diseases such as viral hog cholera, foot-and-mouth disease, and rabies. Vaccines and antibody to bacterial diseases such as diphtheria (Corynebacterium diphtheriae), scarlet fever (Streptococcus pyogenes), tuberculosis (Mycobacterium tuberculosis), and whooping cough (Bordetella pertussis), and typhoid (Salmonella typhi) and virus diseases such as small pox (Variola major, Variola minor), measles (rubeola), German measles (rubella), mumps (epidemic parotitis), influenza, and polyomyelitis may be manufactured by the process or the processes of the present invention. It should be noted that vaccines described here are free of side effects which have limited general use of certain prior art vaccines. For example, according to the present invention a vaccine which consists essentially of a synthetic peptide whose sequence is derived from DNA sequencing, which includes the specific antigenic determinant for a particular strain of influenza on a carrier and which is free of the side effects which have limited use of prior art influenza vaccines is now possible. Broad protection, without adverse side effects, from influenza can be obained by preparing vaccines as described herein against the more common or expected strains of influenza inducing virus. The manufacture of vaccines and antibody preparations for prevention and diagnosis of above bacterial and viral diseases are, of course, merely exemplary of the general scope and applicability of this invention.

Fungi, yeast, somatic cells

The concept and processes of this invention are also applicable to the prevention of pathogenic reaction to other organisms, such as fungi, yeasts and pathogenic somatic cell fragments, in which an antibody-antigen binding occurs to inactivate the organism or to neutralize or prevent the pathogenic reaction of the organism in the host. The very same procedure is applied to fungi and yeasts; namely, the portion of the organism genome that encodes the polar protein indentified. The gene of interest is then cloned and its nucleotide sequence is determined. The nucleotide sequence is then used to determine the amino acid sequence of the probable antigen protein, and a large number of potential antigenic determinants are chemically synthesized, coupled to a carrier and injected into a suitable host. The immunological response for each such "antigen" is determined and the particular, specific antigenic determinant of immunological interest is identified as associated with a particular antigen and having produced antibodies to the organism and which does not react adversely to the host. The thus identified specific antigenic determinant containing peptide is then chemically synthesized in such quantity as may be desired and the vaccine produced by binding the peptide to a carrier is used to protect a host from the organism's pathogenicity. Oligospecific antibodies for diagnostic or other purposes may also be harvested.

Example—Diagnostic techniques

The method of the invention may be used, for example, in the preparation of diagnostic tests, such as immunoassays, in which it is necessary to have antibodies to the organism to be detected or a synthetic antigen mimicking a determinant on the organism to be detected. Such diagnostic techniques include, for example, enzyme immune assay, radioimmune assay, fluorescence immune assay, and other techniques in which either the antibody or the antigen is labelled with some detectable tag.

Thus, using the double antibody technique outlined by Voller, et al., "Enzyme Immune Assays in Diagnostic Medicine", *Bulletin of the World Health Organization,* Volume 53, pp. 55—65 (1976), an ELISA Test was conducted to determine the applicability of the method of the invention to preparation of diagnostic tests. Anti-R serum and the R pentadecapeptide antigen (obtained as described above) were employed. Some of the antibody was conjugated with horseradish peroxidase enzyme by the periodide method of Nakana and Kawaoi, *Journal of Histochemistry and Cytochemistry,* Volume 22, pp. 1084—1091 (1974). Following known enzyme immune assay techniques, a Linbro plate was coated with purified antibody and washed. The R protein was then added to certain of the wells of the plate, followed by an incubation period and a second wash. Finally, the antibody peroxides conjugate was added to all of the wells followed by a second incubation and a third wash. Addition of substrate to the wells gave a positive response (color) in those wells to which antigen had been added but not in those wells to which antigen had not been added, thus demonstrating the practicality of the test.

In a like way, other immunoassay tests may be conducted using products made following the method of the present invention.

Example—Passive immunization

For many diseases, protein is obtained by injecting a vaccine (containing a debilitated organism) into the subject to be protected, whereupon the subject generates antibodies to the debilitated organism in the vaccine. While this technique is practiced for many diseases, there are other diseases for which the preferred route of protection is the injection of antibody to the disease rather than injection of a material which will cause production of this antibody by the subject. This type of immunization is referred to as "passive immunization".

In cases where passive immunization is desired, the method of the present invention may be utilized to produce antibodies to determinants on the respective disease organism, which antibodies may then be used for passive immunization.

The method of the present invention is particularly useful with regard to the preparation of vaccines for diseases which are either dangerous to handle or difficult to grow, such as hepatitis B, rabies, yellow fever, and various rikettsias (e.g., Q fever).

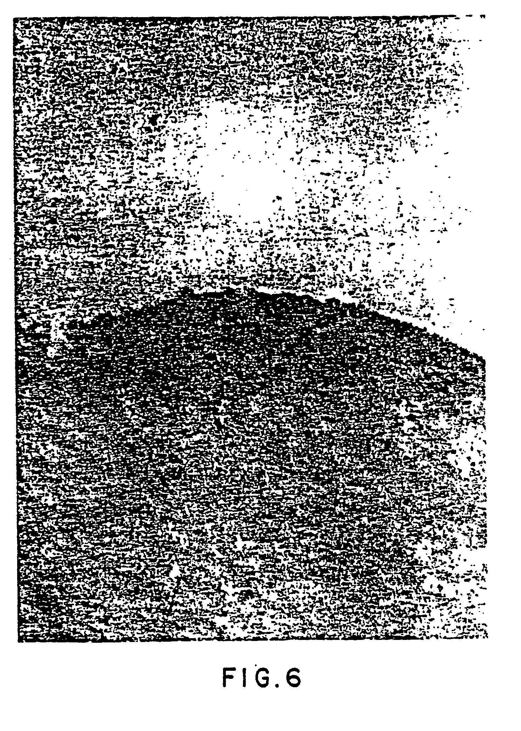

Antibodies produced against R-pentadecapeptide antigen produced as described have been conjugated with ferritin (Singer, S. J. and Schick, A. F., *J. Biophys. Biochem. Cytol.* 9:519 (1961)), and reacted to bind the antigenic sites on virus particles, thereby passivating the virus. The surface of a virus particle with the antigenic sites bound by synthetic antigen induced antibodies visualized by ferritin configuration is shown in the photograph of Figure 6.

In like manner, a vaccine to an organism which induces the production of antigen in the host is manufactured according to this invention. The genoma portion which encodes the production of the antigen in the host is identified by genetic techniques. More than one portion may be tentatively identified, and each portion carried through to final proof as to which portion actually encodes the antigenic determinant of the induced antigen. Each gene of interest is then cloned and its nucleotide sequence determined from which the amino acid sequence of the peptide of interest is determined through conventional genetic coding. The respective peptides are synthesized and bound to carriers to form potential antigens in which the antigenic determinant is the peptide chain. These antigens are introduced into the host and the antigen which initiates production of antibodies to the induced antigen is identified by conventional immunochemical methods. Upon injection of this antigen into a host, antibodies are produced which bind and inactivate the pathogenic antigen produced by, but not expressed in, the organism and prevent or limit the pathogenic effect on the host. The antibodies may, of course be harvested. An example of this application of the present invention is the manufacture of vaccine for the prevention of cancer induced by the Rous sarcoma virus and the preparation of diagnostic oligospecific antibody.

Other pathogens, including chemical pathogenic agents, may also induce the production in the host of antigenic proteins. The present invention encompasses the identification of that gene, whether in a pathogenic organism or in the host, which encodes the antigenic protein, sequencing the gene, synthesizing the peptide which includes the specific antigenic determinant and preparing the antigen as described herein.

It will be readily understood that there are certain to be other applications of the present invention to the preparation of vaccines to diseases in which the specific antigenic determinant does not express itself in the pathogenic organism per se as more such organisms are identified and understood.

Industrial application

The ability to produce antigens which induce one and only one immunological response, the production of antibodies to a pathogen, and to produce antibodies which bind and inactivate only one antigen determinant is of enormous industrial and economic importance in animal husbandry, for example, not to speak of the importance of these techniques in the lives of individual human beings. The present invention finds application in the preparation of vaccines for animals, and also for humans, and in the preparation of antibodies for diagnosis and treatment of animal and human disease.

**Claims for the Contacting States: BE CH DE FR GB IT LI NL SE**

1. A chemically synthesized antigenic peptide containing an amino acid sequence substantially corresponding to an antigenic amino acid sequence present in a natural, pathogenic related protein, said peptide when linked to a carrier and introduced into a host animal being capable of eliciting the production of antibodies that react with said natural pathogenic related protein, and said peptide, taken from left to right and in the direction from amino-terminus to carboxy-terminus, being represented by a formula selected from the group consisting of:

(a) LeuThrGlnGlnPheHisGlnLeuLysProlleGluCysGluPro;

(b) IleLeuAsnArgLeuValGlnPheValLysAspArglleSerValValGlnAlaLeuValLeuThr GlnGlnPheHisGlnLeuLysProlleGluCysGluPro;

(c) PheProGlySerSerThrThrSerThrGlyProCysArgThrCysMetThrThrAlaGlnGlyThr SerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValPro AsnGlyThrLeuValLysThrlleThrAsnAspGlnlleGlu;

(f) AsnAlaThrGluLeuValGlnSerSerSerThrGlyLyslleCysAsnAsnProHisArglleLeu AspGlylleAsnCys;

(g) CysAsnAsnProHisArglleLeuAspGlylleAsnCysThrLeulleAspAlaLeuLeuGlyAsp ProHisCysAspGlyPheGlnAsnGluLysTrpAspLeuPhe;

(h) AspTyrAlaSerLeuArgSerLeuValAlaSerSerGlyThrLeuGluPhelleAsnGluGlyPhe AsnTrpThrGlyValThrGlnAsnGlyGlySerSerAlaCys;

(i) SerGlyLysValThrValSerThrLysArgSerGlnGlnThrllelleProAsnValGlySerArg ProTrpValArgGlyLeu;

(j) CysProLysTyrValLysGlnAsnThrLeuLysLeuAlaThrGlyMetArgAsnValProGluLys GlnThrArg;

(k) GlnAspLeuProGlyAsnAspAsnAsnSerThrAlaThrLeuCys;

(l) CysLeuGlyHisHisAlaValProAsnGlyThrLeuValLysThrlleThrAsnAspGlnlleGlu ValThrAsnAlaThrGluLeuValGlnSerSerSerThrGlyLyslleCys;

(m) CysAsnAsnProHisArglleLeuAspGlylleAsnCys;

(n) CysAsnAsnProHisArglleLeu;

(o) HisCysAspGlyPheGlnAsnGluLysTrpAspLeuPheValGlu;

(p) HisCysAspGlyPheGlnAsnGluLysTrpAspLeuPheValGluArgSerLysAlaPheSerAsn CysTyrProTyrAspValProAspTyrAlaSerLeuArgSer;

(q) ValThrGlnAsnGlyGlySerSerAlaCysLysArgGlyProAspSer;

(r) CysLysArgGlyProAspSerLysArgGlyProAspSerGlyPhePheSerArgLeuAsnTrpLeuTyr;

(s) CysLysArgGlyProAspSerLysArgGlyProAspSerGlyPhePheSerArgLeuAsnTrpLeu TyrLysSerGlySerTrpTyrProValGlnAsnValTrpMetProAsnAsnAsp AsnSer;

(t) AsnSerAspLysLeuTyrlleTrpGlyValHisHisProSerThrAspLysGluGlnThrAsnLeu TyrVal;

(u) HisHisProSerThrAspLysGluGlnThrAsnLeuTyrVal;

(z) CysLeuGlyGlnAsnSerGlnSerProThrSerAsnHisSerProThrSerCysProProThrCys ProGlyTyrArgTrpMetCysLeuArgArgPhelle;

(aa) GluAsnlleThrSerGlyPheLeuGlyProLeuLeuValLeuGln;

(bb) LeuThrArglleLeuThrlleProGlnSerLeuAspSerTrp;

(ee) LeuValLeuLeuAspTyrGlnGlyMetLeuProValCysProLeu; and

(ff) any peptide comprising at least a sequence of 6 or more amino acids selected from the sequence of (a) through (ee) above.

2. A synthetic antigen consisting essentially of a synthetic peptide according to claim 1 linked to a carrier.

3. A synthetic antigen according to claim 2 wherein the carrier is a lipid moiety.

4. A synthetic antigen consisting essentially of a plurality of synthetic peptides according to claim 1 linked to a carrier.

5. An antigen consisting essentially of a carrier linked to one or more synthetic peptides of claim 1, said one or more peptides, taken from left to right and in the direction from amino-terminus to carboxy-terminus, being represented by a formula selected from the group consisting of:

    (a)  LeuThrGlnGlnPheHisGlnLeuLysProIleGluCysGluPro;

    (b)  IleLeuAsnArgLeuValGlnPheValLysAspArgIleSerValValGlnAlaLeuValLeuThr
GlnGlnPheHisGlnLeuLysProIleGluCysGluPro;

    (c)  PheProGlySerSerThrThrSerThrGlyProCysArgThrCysMetThrThrAlaGlnGlyThr
SerMetTyrProSerCys;

    (d)  PheProGlySerSerThrThrSerThrGlyProCysArgThr;

    (e)  GlnAspLeuProGlyAsnAspAsnAsnSerThrAlaThrLeuCysLeuGlyHisHisAlaValPro
AsnGlyThrLeuValLysThrIleThrAsnAspGlnIleGlu;

    (f)  AsnAlaThrGluLeuValGlnSerSerSerThrGlyLysIleCysAsnAsnProHisArgIleLeu
AspGlyIleAsnCys;

    (g)  CysAsnAsnProHisArgIleLeuAspGlyIleAsnCysThrLeuIleAspAlaLeuLeuGlyAsp
ProHisCysAspGlyPheGlnAsnGluLysTrpAspLeuPhe;

    (h)  AspTyrAlaSerLeuArgSerLeuValAlaSerSerGlyThrLeuGluPheIleAsnGluGlyPhe
AsnTrpThrGlyValThrGlnAsnGlyGlySerSerAlaCys;

    (i)  SerGlyLysValThrValSerThrLysArgSerGlnGlnThrIleIleProAsnValGlySerArg
ProTrpValArgGlyLeu;

    (j)  CysProLysTyrValLysGlnAsnThrLeuLysLeuAlaThrGlyMetArgAsnValProGluLys
GlnThrArg;

    (k)  any peptide comprising at least a sequence of 6 or more amino acids selected from the sequence of (a) through (j) above.

6. An antigenic peptide according to claim 1 or a synthetic antigen according to any of claims 2 to 5 for use in the treatment, prevention or diagnosis of infections by pathogenic organisms.

7. A vaccine to an antigenic protein induced in an animal host by a pathogen comprising antigen of any of claims 1 to 5 in an amount sufficient to elicit production of antibodies in said host and to protect said host.

8. A preparation of antibodies elicited in and harvested from a host in response to the introduction into said host of an effective amount of the synthetic antigen according to any of claims 1 to 5, said antibodies being capable of reacting with said natural, pathogen related protein and protecting the host.

9. A method for manufacturing a preparation of antibodies that react with a natural, animal pathogen related protein and protect an animal host comprising the steps of:

(i) providing the synthetic antigen of claim 2;

(ii) introducing into a host animal an aliquot of said antigen in an amount sufficient to elicit production of antibodies in said host;

(iii) assaying the produced antibodies for capability to react with said natural, pathogen related protein and to protect the animal host;

(iv) making larger quantities than by steps (i) and (ii) of said antibodies that react with the natural, pathogen related protein and protect the animal host from said pathogen; and

(v) harvesting said antibodies from the host.

**Claims for the Contracting State: AT**

1. A method of obtaining an antigenic peptide containing an amino acid sequence substantially corresponding to an antigenic amino acid sequence present in a natural, pathogen related protein, said peptide when linked to a carrier and introduced into a host animal being capable of eliciting the production of antibodies that react with said natural pathogen related protein, said method comprising synthesizing a peptide being represented by a formula selected from the group consisting of:

    (a)    LeuThrGlnGlnPheHisGlnLeuLysProIleGluCysGluPro;

    (b)    IleLeuAsnArgLeuValGlnPheValLysAspArgIleSerValValGlnAlaLeuValLeuThr
GlnGlnPheHisGlnLeuLysProIleGluCysGluPro;

    (c)    PheProGlySerSerThrThrSerThrGlyProCysArgThrCysMetThrThrAlaGlnGlyThr
SerMetTyrProSerCys;

    (d)    PheProGlySerSerThrThrSerThrGlyProCysArgThr;

    (e)    GlnAspLeuProGlyAsnAspAsnAsnSerThrAlaThrLeuCysLeuGlyHisHisAlaValPro
AsnGlyThrLeuValLysThrIleThrAsnAspGlnIleGlu;

    (f)    AsnAlaThrGluLeuValGlnSerSerSerThrGlyLysIleCysAsnAsnProHisArgIleLeu
AspGlyIleAsnCys;

    (g)    CysAsnAsnProHisArgIleLeuAspGlyIleAsnCysThrLeuIleAspAlaLeuLeuGlyAsp
ProHisCysAspGlyPheGlnAsnGluLysTrpAspLeuPhe;

(h)   AspTyrAlaSerLeuArgSerLeuValAlaSerSerGlyThrLeuGluPheIleAsnGluGlyPhe
AsnTrpThrGlyValThrGlnAsnGlyGlySerSerAlaCys;

(i)   SerGlyLysValThrValSerThrLysArgSerGlnGlnThrIleIleProAsnValGlySerArg
ProTrpValArgGlyLeu;

(j)   CysProLysTyrValLysGlnAsnThrLeuLysLeuAlaThrGlyMetArgAsnValProGluLys
GlnThrArg;

(k)   GlnAspLeuProGlyAsnAspAsnAsnSerThrAlaThrLeuCys;

(l)   CysLeuGlyHisHisAlaValProAsnGlyThrLeuValLysThrIleThrAsnASspGlnIleGlu
ValThrAsnAlaThrGluLeuValGlnSerSerSerThrGlyLysIleCys;

(m)   CysAsnAsnProHisArgIleLeuAspGlyIleAsnCys;

(n)   CysAsnAsnProHisArgIleLeu;

(o)   HisCysAspGlyPheGlnAsnGluLysTrpAspLeuPheValGlu;

(p)   HisCysAspGlyPheGlnAsnGluLysTrpAspLeuPheValGluArgSerLysAlaPheSerAsn
CysTyrProTyrAspValProAspTyrAlaSerLeuArgSer;

(q)   ValThrGlnAsnGlyGlySerSerAlaCysLysArgGlyProAspSer;

(r)   CysLysArgGlyProAspSerLysArgGlyProAspSerGlyPhePheSerArgLeuAsnTrpLeuTyr;

(s)   CysLysArgGlyProAspSerLysArgGlyProAspSerGlyPhePheSerArgLeuAsnTrpLeu
TyrLysSerGlySerTrpTyrProValGlnAsnValTrpMetProAsnAsnAsp
AsnSer;

(t)   AsnSerAspLysLeuTyrIleTrpGlyValHisHisProSerThrAspLysGluGlnThrAsnLeu
TyrVal;

(u)   HisHisProSerThrAspLysGluGlnThrAsnLeuTyrVal;

(z)   CysLeuGlyGlnAsnSerGlnSerProThrSerAsnHisSerProThrSerCysProProThrCys
ProGlyTyrArgTrpMetCysLeuArgArgPheIle;

(aa)   GluAsnIleThrSerGlyPheLeuGlyProLeuLeuValLeuGln;

(bb)   LeuThrArgIleLeuThrIleProGlnSerLeuAspSerTrp;

(ee)   LeuValLeuLeuAspTyrtGlnGlyMetLeuProValCysProLeu; and

(ff)   any peptide comprising at least a sequence of 6 or more amino acids selected from the sequence
of (a) through (ee) above.

2. A method of obtaining a synthetic antigen comprising linking a synthetic antigenic peptide, obtained by a method according to claim 1 to a carrier.

3. A method according to claim 2 wherein the carrier is a lipid moiety.

4. A method according to claim 2 comprising linking a plurality of said synthetic antigenic peptides to said carrier.

5. A method of obtaining a synthetic antigen comprising linking to a carrier one or more peptides being represented by a formula selected from the group consisting of:

(a)   LeuThrGlnGlnPheHisGlnLeuLysProIleGluCysGluPro;

(b)   IleLeuAsnArgLeuValGlnPheValLysAspArgIleSerValValGlnAlaLeuValLeuThr
GlnGlnPheHisGlnLeuLysProIleGluCysGluPro;

(c)   PheProGlySerSerThrThrSerThrGlyProCysArgThrCysMetThrThrAlaGlnGlyThr
SerMetTyrProSerCys;

(d)   PheProGlySerSerThrThrSerThrGlyProCysArgThr;

(e)   GlnAspLeuProGlyAsnAspAsnAsnSerThrAlaThrLeuCysLeuGlyHisHisAlaValPro
AsnGlyThrLeuValLysThrIleThrAsnAspGlnIleGlu;

(f)   AsnAlaThrGluLeuValGlnSerSerSerThrGlyLysIleCysAsnAsnProHisArgIleLeu
AspGlyIleAsnCys;

(g)   CysAsnAsnProHisArgIleLeuAspGlyIleAsnCysThrLeuIleAspAlaLeuLeuGlyAsp
ProHisCysAspGlyPheGlnAsnGluLysTrpAspLeuPhe;

(h)   AspTyrAlaSerLeuArgSerLeuValAlaSerSerGlyThrLeuGluPheIleAsnGluGlyPhe
AsnTrpThrGlyValThrGlnAsnGlyGlySerSerAlaCys;

(i)   SerGlyLysValThrValSerThrLysArgSerGlnGlnThrIleIleProAsnValGlySerArg
ProTrpValArgGlyLeu;

(j)   CysProLysTyrValLysGlnAsnThrLeuLysLeuAlaThrGlyMetArgAsnValProGluLys
GlnThrArg;

(k)   any peptide comprising at least a sequence of 6 or more amino acids selected from the sequence
of (a) through (j) above.

6. A method for manufacturing a preparation of antibodies that react with a natural, animal pathogen related protein and protect an animal host comprising the steps of:

(i) providing the synthetic antigen of claim 2;

(ii) introducing into a host animal an aliquot of said antigen in an amount sufficient to elicit production of antibodies in said host;

(iii) assaying the produced antibodies for capability to react with said natural, pathogen related protein and to protect the animal host;

(iv) making larger quantities than by steps (i) and (ii) of said antibodies that react with the natural, pathogen related protein and protect the animal host from said pathogen; and

27

(v) harvesting said antibodies from the host.


**Patentansprüche für die Vertragsstaaten: BE CH DE FR GB IT LI NL SE**

1. Chemisch synthetisiertes Antigen-Peptid mit einer Aminosäuresequenz, die im wesentlichen einer in einem natürlichen, Pathogen-verwandten Protein vorliegenden Antigen-Aminosäuresequenz entspricht, welches Peptid, bei Bindung an einen Träger und Einführung in ein Wirtstier, befähigt ist, die Produktion von Antikörpern hervorzurufen, welche mit dem genannten natürlichen, Pathogen-verwandten Protein reagieren, und welches Peptid, won links nach rechts und in der Richtung von der Aminoendgruppe zur Carboxyendruppe gesehen, durch eine Formel dargestellt wird, die aus einer Gruppe ausgewählt wird, die aus:

(a) LeuThrGlnGlnPheHisGlnLeuLysProIleGluCysGluPro;

(b) IleLeuAsnArgLeuValGlnPheValLysAspArgIleSerValValGlnAlaLeuValLeuThrGlnGlnPheHisGlnLeu-LysProIleGluCysGluPro;

(c) PheProGlySerSerThrThrSerThrGlyProCysArgThrCysMetThrThrAlaGlnGlyThrSerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValProAsnGlyThrLeuVal-LysThrIleThrAsnAspGlnIleGlu;

(f) AsnAlaThrGluLeuValGlnSerSerSerThrGlyLysIleCysAsnAsnProHisArgIleLeuAspGlyIleAsnCys;

(g) CysAsnAsnProHisArgIleLeuAspGlyIleAsnCysThrLeuIleAspAlaLeuLeuGlyAspProHisCysAspGlyPhe-GlnAsnGluLysTrpAspLeuPhe;

(h) AspTyrAlaSerLeuArgSerLeuValAlaSerSerGlyThrLeuGluPheIleAsnGluGlyPheAsnTrpThrGlyValThr-GlnAsnGlyGlySerSerAlaCys;

(i) SerGlyLysValThrValSerThrLysArgSerGlnGlnThrIleIleProAsnValGlySerArgProTrpValArgGlyLeu;

(j) CysProLysTyrValLysGlnAsnThrLeuLysLeuLysLeuAlaThrGlyMetArgAsnValProGluLysGlnThrArg;

(k) GlnAspLeuProGlyAsnAspAsnAsnSerThrAlaThrLeuCys;

(l) CysLeuGlyHisHisAlaValProAsnGlyThrLeuValLysThrIleThrAsnAspGlnIleGluValThrAsnAlaThrGlu-LeuValGlnSerSerSerThrGlyLysIleCys;

(m) CysAsnAsnProHisArgIleLeuAspGlyIleAsnCys;

(n) CysAsnAsnProHisArgIleLeu;

(o) HisCysAspGlyPheGlnAsnGluLysTrpAspLeuPheValGlu;

(p) HisCysAspGlyPheGlnAsnGluLysTrpAspLeuPheValGluArgSerLysAlaPheSerAsnCysTyrProTyrAsp-ValProAspTyrAlaSerLeuArgSer;

(q) ValThrGlnAsnGlyGlySerSerAlaCysLysArgGlyProAspSer;

(r) CysLysArgGlyProAspSerLysArgGlyProAspSerGlyPhePheSerArgLeuAsnTrpLeuTyr;

(s) CysLysArgGlyProAspSerLysArgGlyProAspSerGlyPhePheSerArgLeuAsnTrpLeuTyrLysSerGlySer-TrpTyrProValGlnAsnValTrpMetProAsnAsnAspAsnSer;

(t) AsnSerAspLysLeuTryIleTrpGlyValHisHisProSerThrAspLysGluGlnThrAsnLeuTyrVal;

(u) HisHisProSerThrAspLysGluGlnThrAsnLeuTyrVal;

(z) CysLeuGlyGlnAsnSerGlnSerProThrSerAsnHisSerProThrSerCysProProThrCysProGlyTyrArgTrpMet-CysLeuArgArgPheIle;

(aa) GluAsnIleThrSerGlyPheLeuGlyProLeuLeuValLeuGln;

(bb) LeuThrArgIleLeuThrIleProGlnSerLeuAspSerTrp;

(ee) LeuValLeuLeuAspTyrGlnGlyMetLeuProValCysProLeu;

und

(ff) einem beliebigen Peptid, umfassend wenigstens eine Sequenz von 6 oder Mehr Aminosäuren, ausgewählt aus der vorstehenden Sequenz von (a) bis (ee), besteht.

2. Synthetisches Antigen, im wesentlichen bestehend aus einem an einen Träger gebundenen synthetischen Peptid gemäß Anspruch 1.

3. Synthetisches Antigen nach Anspruch 2, worin der Träger ein Lipidrest ist.

4. Synthetisches Antigen, im wesentlichen bestehend aus einer Mehrzahl von an einen Träger gebundenen synthetischen Peptiden nach Anspruch 1.

5. Antigen, im wesentlichen bestehend aus einem an ein oder mehrere synthetische Peptide nach Anspruch 1 gebundenen Tägter, welches eine bzw. welche mehrere Peptide, von links nach rechts und in der Richtung von der Aminoendgruppe zur Carboxyendgruppe gesehen, durch eine Formel dargestellt wird bzw. werden, ausgewählt aus der Gruppe, die aus:

(a) LeuThrGlnGlnPheHisGlnLeuLysProIleGluCysGluPro;

(b) IleLeuAsnArgLeuValGlnPheValLysAspArgIleSerValValGlnAlaLeuValLeuThrGlnGlnPheHisGlnLeu-LysProIleGluCysGluPro;

(c) PheProGlySerSerThrThrSerThrGlyProCysArgThrCysMetThrThrAlaGlnGlyThrSerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValProAsnGlyThrLeuVal-LysThrIleThrAsnAspGlnIleGlu;

(f) AsnAlaThrGluLeuValGlnSerSerSerThrGlyLysIleCysAsnAsnProHisArgIleLeuAspGlyIleAsnCys;

28

(g) CysAsnAsnProHisArgIleLeuAspGlyIleAsnCysThrLeuIleAspAlaLeuLeuGlyAspProHisCysAspGlyPhe-GlnAsnGluLysTrpAspLeuPhe;

(h) AspTyrAlaSerLeuArgSerLeuValAlaSerSerGlyThrLeuGluPheIleAsnGluGlyPheAsnTrpThrGlyValThr-GlnAsnGlyGlySerSerAlaCys;

(i) SerGlyLysValThrValSerThrLysArgSerGlnGlnThrIleIleProAsnValGlySerArgProTrpGalArgGlyLeu;

(j) CysProLysTyrValLysGlnAsnThrLeuLysLeuAlaThrGlyMetArgAsnValProGluLysGlnThrArg; und

(k) einem beliebigen Peptid, umfassend wenigstens eine Sequenz von sechs oder mehr Aminosäuren, ausgewählt aus der vorstehenden Sequenz von (a) bis (j), besteht.

6. Antigen-Peptid nach Anspruch 1 oder synthetisches Antigen nach einem der Ansprüche 2 bis 5 zur Verwendung in der Behandlung, Verhütung oder Diagnose von Infektionen durch pathogene Organismen.

7. Impstoff für eine Antigen-Protein, das in einem Wirtstier durch ein Pathogen ein Pathogen induziert worden ist, umfassend ein Antigen nach einem der Ansprüche 1 bis 5 in einer zur Hervorrufung einer Produktion von Antikörpern in dem genannten Wird und zum Schutz des genannten Wirtes ausreichenden Menge.

8. Antikörperpräparat, das in einem Wirt hervorgerufen und von dem Wirt als Reaktion auf das Einführen einer wirksamen Menge des synthetischen Antigens nach einem der Ansprüche 1 bis 5 in den genannten Wirt geerntet worden ist, welche Antikörper befähigt sind, mit dem genannten, Pathogen-verwandten Protein zu reagieren und den Wirt zu schützen.

9. Verfahren zur Bereitung eines Präparates von Antikörpern, welche mit einem natürlichen, tierischen Pathogen-verwandten Protein reagieren und ein Wirtstier schützen, umfassend die Stufen:

(i) Bereiten des synthetischen Antigens nach Anspruch 2;

(ii) Einführen eines aliquoten Teiles des genannten Antigens in ein Wirtstier in einer Menge, die zur Hervorrufung einer Produktion von Antiköpern in dem genannten Wirt ausreicht;

(iii) Überprufen der gebildeten Antikörper auf ihr Vermögen, mit dem genannten, Pathogen-verwandteten Protein zu reagieren und das Wirtstier zu schützen;

(iv) Herstellen größer Mengen als nach den Stufen (i) und (ii) der genannten Antikörper, die mit dem natürlichen Pathogen-verwandten Protein reagieren und das Wirtstier vor dem genannten Pathogen schützen;

(v) Ernten der genannten Antikörper von dem Wirt.

## Patentansprüche für den Vertragsstaat: AT

1. Verfahren zur Herstellung eines Antigen-Peptid mit einer Aminosäuresequenz, die im wesentlichen einer in einem natürlichen, Pathogen-verwandten Protein vorliegenden Antigen-Aminosäuresequenz entspricht, welches Peptid, bei Bindung an einen Träger und Einführung in ein Wirtstier, befähigt ist, die Produktion von Antikörpern hervorzurufen, welche mit dem genannten natürlichen, Pathogen-verwandten Protein reagieren, welches Verfahren ein Synthetisieren eines Peptids umfaßt, das durch eine Formel dargestellt wird, die aus einer Gruppe ausgewält wird, die aus:

HG (a) LeuThrGlnGlnPheHisGlnLeuLysProIleGluCysGluPro;

(b) IleLeuAsnArgLeuValGlnPheValLysAspArgIleSerValValGlnAlaLeuValLeuThrGlnGlnPheHisGlnLeu-LysProIleGluCysGluPro;

(c) PheProGlySerSerThrThrSerThrGlyProCysArgThrCysMetThrThrAlaGlnGlyThrSerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValProAsnGlyThrLeuVal-LysThrIleThrAsnAspGlnIleIleGlu;

(f) AsnAlaThrGluLeuValGlnSerSerSerThrGlyLysIleCysAsnAsnProHisArgIleLeuAspGlyIleAsnCys;

(g) CysAsnAsnProHisArgIleLeuAspGlyIleAsnCysThrLeuIleAspAlaLeuLeuGlyAspProHisCysAspGlyPhe-GlnAsnGluLysTrpAspLeuPhe;

(h) AspTyrAlaSerLeuArgSerLeuValAlaSerSerGlyThrLeuGluPheIleAsnGluGlyPheAsnTrpThrGlyValThr-GlnAsnGlyGlySerSerAlaCys;

(i) SerGlyLysValThrValSerThrLysArgSerGlnGlnThrIleIleProAsnValGlySerArgProTrpValArgGlyLeu;

(j) CysProLysTyrValLysGlnAsnThrLeuLysLeuLysLeuAlaThrGlyMetArgAsnValProGluLysGlnThrArg;

(k) GlnAspLeuProGlyAsnAspAsnAsnSerThrAlaThrLeuCys;

(l) CysLeuGlyHisHisAlaValProAsnGlyThrLeuValLysThrIleThrAsnAspGlnIleIleGluValThrAsnAlaThrGlu-LeuValGlnSerSerSerThrGlyLysIleCys;

(m) CysAsnAsnProHisArgIleLeuAspGlyIleAsnCys;

(n) CysAsnAsnProHisArgIleLeu;

(o) HisCysAspGlyPheGlnAsnGluLysTrpAspLeuPheValGlu;

(p) HisCysAspGlyPheGlnAsnGluLysTrpAspLeuPheValGluArgSerLysAlaPheSerAsnCysTyrProTyrAsp-ValProAspTyrAlaSerLeuArgSer;

(q) ValThrGlnAsnGlyGlySerSerAlaCysLysArgGlyProAspSer;

(r) CysLysArgGlyProAspSerLysArgGlyProAspSerGlyPhePheSerArgLeuAsnTrpLeuTyr;

(s) CysLysArgGlyProAspSerLysArgGlyProAspSerGlyPhePheSerArgLeuAsnTrpLeuTyrLysSerGlySer-TrpTyrProValGlnAsnValTrpMetProAsnAsnAspAsnSer;

(t) AsnSerAspLysLeuTrpIleTrpGlyValHisHisProSerThrAspLysGluGlnThrAsnLeuTyrVal;

(u) HisHisProSerThrAspLysGluGlnThrAsnLeuTyrVal;

(z) CysLeuGlyGlnAsnSerGlnSerProThrSerAsnHisSerProThrSerCysProProThrCysProGlyTyrArgTrpMet-CysLeuArgArgPheIle;

(aa) GluAsnIleThrSerGlyPheLeuGlyProLeuLeuValLeuGln;

(bb) LeuThrArgIleLeuThrIleProGlnSerLeuAspSerTrp;

(ee) LeuValLeuLeuAspTyrGlnGlyMetLeuProValCysProLeu;

und

(ff) einem beliebigen Peptid, umfassend wenigstens eine Sequenz von 6 oder Mehr Aminosäuren, ausgewählt aus der vorstehenden Sequenz von (a) bis (ee), besteht.

2. Verfahren zur Herstellung eines synthetischen Antigens, umfassend das Binden eines synthetischen Antigen-Peptids, erhalten nach einem Verfahren gemäß Anspruch 1, an einen Träger.

3. Verfahren nach nach Anspruch 2, worin der Träger ein Lipidrest ist.

4. Verfahren nach nach Anspruch 2, umfassend das Binden einer Mehrzahl von den genannten synthetischen Antigen-Peptiden an den genannten Träger.

5. Verfahren zur Herstellung eines synthetischen Antigens, umfassend das Binden eines oder mehrerer Peptide an einen Träger, welche Peptide durch eine Formel dargestellt werden, ausgewählt aus der Gruppe, die aus:

(a) LeuThrGlnGlnPheHisGlnLeuLysProIleGluCysGluPro;

(b) IleLeuAsnArgLeuValGlnPheValLysAspArgIleSerValValGlnAlaLeuValLeuThrGlnGlnPheHisGlnLeu-LysProIleGluCysGluPro;

(c) PheProGlySerSerThrThrSerThrGlyProCysArgThrCysMetThrThrAlaGlnGlyThrSerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValProAsnGlyThrLeuVal-LysThrIleThrAsnAspGlnIleGlu;

(f) AsnAlaThrGluLeuValGlnSerSerSerThrGlyLysIleCysAsnAsnProHisArgIleLeuAspGlyIleAsnCys;

(g) CysAsnAsnProHisArgIleLeuAspGlyIleAsnCysThrLeuIleAspAlaLeuLeuGlyAspProHisCysAspGlyPhe-GlnAsnGluLysTrpAspLeuPhe;

(h) AspTyrAlaSerLeuSerLeuValAlaSerSerGlyThrLeuGluPheIleAsnGluGlyPheAsnTrpThrGlyValThrGln-AsnGlyGlySerSerAlaCys;

(i) SerGlyLysValThrValSerThrLysArgSerGlnGlnThrIleIleProAsnValGlySerArgProTrpValArgGlyLeu;

(j) CysProLysTyrValLysGlnAsnThrLeuLysLeuLysLeuAlaThrGlyMetArgAsnValProGluLysThrArg; und

(k) einem beliebigen Peptid, umfassend wenigstens eine Sequenz von 6 oder mehreren Aminosäuren, ausgewählt aus der vorstehenden Sequenz von (a) bis (j), besteht.

6. Verfahren zur Bereitung eines Präparats von Antikörpern, welche mit einem natürlichen, tierischen Pathogen-verwandten Proten reagieren und ein Wirtstier schützen, umfassend die Stufen:

(i) Bereiten des synthetischen Antigens nach Anspruch 2;

(ii) Einführen eines aliquoten Teiles des genannten Antigens in ein Wirtstien in einer Menge, die zur Hervorrufung einer Produktion von Antikörpern in dem genannten Wirt ausreicht;

(iii) Überprufen der gebildeten Antikörper auf ihr Vermögen, mit dem genannten, Pathogenverwandeten Protein zu reagieren und das Wirtstier zu schützen;

(iv) Herstellen größere Mengen als nach den Stufen (i) und (ii) der genannten Antikörper, die mit dem natürlichen Pathogen-verwandten Protein reagieren und das Wirtstier vor dem genannten Pathogen schützen;

und

(v) Ernten der genannten Antikörper von dem Wirt.

**Revendications par les Etats contractants: BE CH DE FR GB IT LI NL SE**

1. Peptide antigénique chimiquement synthétisé contenant une séquence d'acides aminés correspondant sensiblement à une séquence d'acides aminés antigéniques présente dans une protéine naturelle en rapport avec le pathogène, ledit peptide lorsqu'il est lié à un support et introduit dans un animal hôte étant capable d'éliciter la production des anticorps qui réagissent avec ladite protéine naturelle en rapport avec le pathogène, et ledit peptide, pris de la gauche à la droite et dans la direction de l'amino-terminal au carboxy-terminal, étant représenté par une formule choisie dans le groupe consistant en:

(a) LeuThrGlnGlnPheHisGlnLeuLysProIleGluCysGluPro;

(b) IleLeuAsnArgLeuValGlnPheValLysAspArgIleSerValValGlnAlaLeuValLeuThrGlnGlnPheHisGlnLeu-LysProIleGluCysGluPro;

(c) PheProGlySerSerThrThrSerThrGlyProCysArgThrCysMetThrThrAlaGlnGlyThrSerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValProAsnGlyThrLeuVal-LysThrIleThrAsnAspGlnIleGlu;

(f) AsnAlaThrGluLeuValGlnSerSerSerThrGlyLysIleCysAsnAsnProHisArgIleLeuAspGlyIleAsnCys;

(g) CysAsnAsnProHisArgIleLeuAspGlyIleAsnCysThrLeuIleAspAlaLeuLeuGlyAspProHisCysAspGlyPhe-GlnAsnGluLysTrpAspLeuPhe;

(h) AspTyrAlaSerLeuArgSerLeuValAlaSerSerGlyThrLeuGluPheIleAsnGluGlyPheAsnTrpThrGlyValThr-GlnAsnGlyGlySerSerAlaCys;

(i) SerGlyLysValThrValSerThrLysArgSerGlnGlnThrIleIleProAsnValGlySerArgProTrpValArgGlyLeu;

(j) CysProLysTyrValLysGlnAsnThrLeuLysLeuLysLeuAlaThrGlyMetArgAsnValProGluLysGlnThrArg;

(k) GlnAspLeuProGlyAsnAspAsnAsnSerThrAlaThrLeuCys;

(l) CysLeuGlyHisHisAlaValProAsnGlyThrLeuValLysThrIleThrAsnAspGlnIleGluValThrAsnAlaThrGlu-LeuValGlnSerSerSerThrGlyLysIleCys;

(m) CysAsnAsnProHisArgIleLeuAspGlyIleAsnCys;

(n) CysAsnAsnProHisArgIleLeu;

(o) HisCysAspGlyPheGlnAsnGluLysTrpAspLeuPheValGlu;

(p) HisCysAspGlyPheGlnAsnGluLysTrpAspLeuPheValGluArgSerLysAlaPheSerAsnCysTyrProTyrAsp-ValProAspTyrAlaSerLeuArgSer;

(q) ValThrGlnAsnGlyGlySerSerAlaCysLysArgGlyProAspSer;

(r) CysLysArgGlyProAspSerLysArgGlyProAspSerGlyPhePheSerArgLeuAsnTrpLeuTyr;

(s) CysLysArgGlyProAspSerLysArgGlyProAspSerGlyPhePheSerArgLeuAsnTrpLeuTyrLysSerGlySer-TrpTyrProValGlnAsnValTrpMetProAsnAsnAspAsnSer;

(t) AsnSerAspLysLeuTryIleTrpGlyValHisHisProSerThrAspLysGluGlnThrAsnLeuTyrVal;

(u) HisHisProSerThrAspLysGluGlnThrAsnLeuTyrVal;

(z) CysLeuGlyGlnAsnSerGlnSerProThrSerAsnHisSerProThrSerCysProProThrCysProGlyTyrArgTrpMet-CysLeuArgArgPheIle;

(aa) GluAsnIleThrSerGlyPheLeuGlyProLeuLeuValLeuGln;

(bb) LeuThrArgIleLeuThrIleProGlnSerLeuAspSerTrp;

(ee) LeuValLeuLeuAspTyrGlnGlyMetLeuProValCysProLeu;

(ff) Tout peptide comprenant au moins une séquence de 6 acides aminés ou plus choisie dans la séquence de (a) à (ee) ci-dessus.

2. Antigène synthétique consistant essentiellement en un peptide synthétique selon la revendication 1 lié à un support.

3. Antigène synthétique selon la revendication 2 où le support est un fragment de lipide.

4. Antigène synthétique consistant essentiellement en un certain nombre de peptides synthétiques selon la revendication 1 liés à un support.

5. Antigène consistant essentiellement en un support lié à un ou plusieurs peptides synthétiques selon la revendication 1, lesdites peptides, pris de la gauche à la droite et dans la direction de l'amino-terminal au carboxy-terminal, étant représentés par une formule choisie dans le groupe consistant en:

(a) LeuThrGlnGlnPheHisGlnLeuLysProIleGluCysGluPro;

(b) IleLeuAsnArgLeuValGlnPheValLysAspArgIleSerValValGlnAlaLeuValLeuThrGlnGlnPheHisGlnLeu-LysProIleGluCysGluPro;

(c) PheProGlySerSerThrThrSerThrGlyProCysArgThrCysMetThrThrAlaGlnGlyThrSerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValProAsnGlyThrLeuVal-LysThrIleThrAsnAspGlnIleGlu;

(f) AsnAlaThrGluLeuValGlnSerSerSerThrGlyLysIleCysAsnAsnProHisArgIleLeuAspGlyIleAsnCys;

(g) CysAsnAsnProHisArgIleLeuAspGlyIleAsnCysThrLeuIleAspAlaLeuLeuGlyAspProHisCysAspGlyPhe-GlnAsnGluLysTrpAspLeuPhe;

(h) AspTyrAlaSerLeuArgSerLeuValAlaSerSerGlyThrLeuGluPheIleAsnGluGlyPheAsnTrpThrGlyValThr-GlnAsnGlyGlySerSerAlaCys;

(i) SerGlyLysValThrValSerThrLysArgSerGlnGlnThrIleIleProAsnValGlySerArgProTrpValArgGlyLeu;

(j) CysProLysTyrValLysGlnAsnThrLeuLysLeuLysLeuAlaThrGlyMetArgAsnValProGluLysThrArg; et

(k) tout peptide comprenant au moins une séquence de 6 acides aminés ou plus choisie parmi les séquences de (a) à (j) ci-dessus.

6. Peptide antigénique selon la revendication 1 ou un antigène synthétique selon l'une quelconque des revendications 2 à 5 pour une utilisation dans le traitement, la prévention ou le diagnostic d'infections par des organismes pathogènes.

7. Vaccin contre une protéine antigénique induite dans un hôte animal par un pathogène comprenant l'antigène selon l'une quelconque des revendications 1 à 5 en une quantité suffisante pour éliciter la production d'anticorps dans ladit hôte et pour protéger ledit hôté.

8. Préparation d'anticorps éclicités dans et récoltés d'un hôte en réponse à l'introduction, dans ledit hôte, d'une quantité effective de l'antigène synthétique selon l'une quelconque des revendications 1 à 5, lesdits anticorps étant capables de réagir avec ladite protéine naturelle en rapport avec le pathogène et de protéger l'hôte.

9. Procédé de fabrication d'une préparation d'anticorps qui réagissent avec une protéine naturelle en rapport avec un pathogène animal et protègent un hôte animal comprenant les étapes de:

(i) produire l'antigène synthétique de la revendication 2;

(ii) introduire dans un animal hôte une portion dudit antigène en une quantité suffisant pour éliciter la production des anticorps dans ledit hôte;

EP 0 044 710 B2

(iii) déterminer les anticorps produits pour la capacité de réagir avec ladit protéine naturelle en rapport avec le pathogène et pour protéger l'animal hôtre;

(iv) produire de plus grandes quantités qu'aux étapes (i) et (ii) desdits anticorps qui réagissent avec la protéine naturelle en rapport avec la pathogène et protègent l'animal hôte dudit pathogène; et

(v) récolter lesdits anticorps de l'hôte.

**Revendications par l'Etat contractant: AT**

1. Méthode pour obtenir un peptide antigénique contenant une séquence d'acides aminés correspondant sensiblement à une séquence d'acides aminés antigéniques présente dans une protéine naturelle en rapport avec le pathogène, ledit peptide lorsqu'il est lié à un support et introduit dans un animal hôte étant capable d'éliciter la production des anticorps qui réagissent avec ladite protéine naturelle en rapport avec le pathogène, ladite méthode comprenant la synthèse d'un peptide représenté par une formule choisie dans le groupe consistant en:

(a) LeuThrGlnGlnPheHisGlnLeuLysProIleGluCysGluPro;

(b) IleLeuAsnArgLeuValGlnPheValLysAspArgIleSerValValGlnAlaLeuValLeuThrGlnGlnPheHisGlnLeu-LysProIleGluCysGluPro;

(c) PheProGlySerSerThrThrSerThrGlyProCysArgThrCysMetThrThrAlaGlnGlyThrSerMetTyrProSerCys;

(d) PheProGlySerSerThrThrSerThrGlyProCysArgThr;

(e)   GlnAspLeuProGlyAsnAspAsnAsnSerThrAlaThrLeuCysLeuGlyHisHisAlaValProAsnGlyThrLeuVal-LysThrIleThrAsnAspGlnIleGlu;

(f) AsnAlaThrGluLeuValGlnSerSerSerThrGlyLysIleCysAsnAsnProHisArgIleLeuAspGlyIleAsnCys;

(g) CysAsnAsnProHisArgIleLeuAspGlyIleAsnCysThrLeuIleAspAlaLeuLeuGlyAspProHisCysAspGlyPhe-GlnAsnGluLysTrpAspLeuPhe;

(h) AspTyrAlaSerLeuArgSerLeuValAlaSerSerGlyThrLeuGluPheIleAsnGluGlyPheAsnTrpThrGlyValThr-GlnAsnGlyGlySerSerAlaCys;

(i) SerGlyLysValThrValSerThrLysArgSerGlnGlnThrIleIleProAsnValGlySerArgProTrpValArgGlyLeu;

(j) CysProLysTyrValLysGlnAsnThrLeuLysLeuLysLeuAlaThrGlyMetArgAsnValProGluLysThrArg;

(k) GlnAspLeuProGlyAsnAspAsnAsnSerThrAlaThrLeuCys;

(l)   CysLeuGlyHisHisAlaValProAsnGlyThrLeuValLysThrIleThrAsnAspGlnIleGluValThrAsnAlaThrGlu-LeuValGlnSerSerSerThrGlyLysIleCys;

(m) CysAsnAsnProHisArgIleLeuAspGlyIleAsnCys;

(n) CysAsnAsnProHisArgIleLeu;

(o) HisCysAspGlyPheGlnAsnGluLysTrpAspLeuPheValGlu;

(p)   HisCysAspGlyPheGlnAsnGluLysTrpAspLeuPheValGluArgSerLysAlaPheSerAsnCysTyrProTyrAsp-ValProAspTyrAlaSerLeuArgSer;

(q) ValThrGlnAsnGlyGlySerSerAlaCysLysArgGlyProAspSer;

(r) CysLysArgGlyProAspSerLysArgGlyProAspSerGlyPhePheSerArgLeuAsnTrpLeuTyr;

(s) CysLysArgGlyProAspSerLysArgGlyProAspSerGlyPhePheSerArgLeuAsnTrpLeuTyrLysSerGlySer-TrpTyrProValGlnAsnValTrpMetProAsnAsnAspAsnSer;

(t) AsnSerAspLysLeuTryIleTrpGlyValHisHisProSerThrAspLysGluGlnThrAsnLeuTyrVal;

(u) HisHisProSerThrAspLysGluGlnThrAsnLeuTyrVal;

(z) CysLeuGlyGlnAsnSerGlnSerProThrSerAsnHisSerProThrSerCysProProThrCysProGlyTyrArgTrpMet-CysLeuArgArgPheIle;

(aa) GluAsnIleThrSerGlyPheLeuGlyProLeuLeuValLeuGln;

(bb) LeThrArgIleLeuThrIleProGlnSerLeuAspSerTrp;

(ee) LeuValLeuLeuAspTyrGlnGlyMetLeuProValCysProLeu;

(ff) Tout peptide comprenant au moins une séquence de 6 acides aminés ou plus choisie dans la séquence de (a) à (ee) ci-dessus.

2. Méthode pour obtenir un antigène synthétique consistant à lier un peptide synthétique antigénique obtenu par une méthode selon la revendication 1 à un support.

3. Méthode selon la revendication 2 où le support est un fragment de lipide.

4. Méthode selon la revendication 2 consistant à lier un certain nombre desdits peptides synthétiques antigéniques audit support.

5. Méthode pour obtenir un antigène synthétique consistant à lier à un support un ou plusieurs peptides représentés par un formule choisi dans le groupe consistant en:

(a) LeuThrGlnGlnPheHisGlnLeuLysProIleGluCysGluPro;

(b) IleLeuAsnArgLeuValGlnPheValLysAspArgIleSerValValGlnAlaLeuValLeuThrGlnGlnPheHisGlnLeu-LysProIleGluCysGluPro;

(c) PheProGlySerSerThrThrSerThrGlyProCysArgThrCysMetThrThrAlaGlnGlyThrSerMetTyrProSerCys;

(d) PheProGlySerSerThrThrSerThrGlyProCysArgThr;

(e) GlnAspLeuProGlyAsnAspAsnAsnSerThrAlaThrLeuCysLeuGlyHisHisAlaValProAsnGlyThrLeuVal-LysThrIleThrAsnAspGlnIleGlu;

(f) AsnAlaThrGluLeuValGlnSerSerSerThrGlyLysIleCysAsnAsnProHisArgIleLeuAspGlyIleAsnCys;

(g) CysAsnAsnProHisArgIleLeuAspGlyIleAsnCysThrLeuIleAspAlaLeuLeuGlyAspProHisCysAspGlyPhe-GlnAsnGluLysTrpAspLeuPhe;

(h) AspTyrAlaSerLeuSerLeuValAlaSerSerGlyThrLeuGluPheIleAsnGluGlyPheAsnTrpThrGlyValThrGln-AsnGlyGlySerSerAlaCys;

(i) SerGlyLysValThrValSerThrLysArgSerGlnGlnThrIleIleProAsnValGlySerArgProTrpValArgGlyLeu;

(j) CysProLysTyrValLysGlnAsnThrLeuLysLeuLysLeuAlaThrGlyMetArgAsnValProGluLysThrArg; et

(k) tout peptide comprenant au moins une séquence de 6 acides aminés ou plus choisie parmi les séquences de (a) à (j) ci-dessus.

6. Procédé de fabrication d'une préparation d'anticorps qui réagissent avec une protéine naturelle en rapport avec un pathogène animal et protègent un hôte animal comprenant les étapes de:

(i) produire l'antigène synthétique de la revendication 2;

(ii) introduire dans un animal hôte une portion dudit antigène en une quantité suffisant pour éliciter la production des anticorps dans ledit hôte;

(iii) déterminer les anticorps produits pour la capacité de réagir avec ladit protéine naturelle en rapport avec le pathogène et pour protéger l'animal hôtre;

(iv) produire de plus grandes quantités qu'aux étapes (i) et (ii) desdits anticorps qui réagissent avec la protéine naturelle en rapport avec lea pathogène et protègent l'animal hôte dudit pathogène; et

(v) récolter lesdits anticorps de l'hôte.

$(NH_2)$GluProValSerLeuThrLeuAlaLeuLeuLeuGlyGlyLeuThrMetGlyGlyIleAlaAlaGlyIleGlyThrGlyThrThrAlaLeuMetAlaThr
5'TTATTGGGTGGACTAACCATGGGGGGAATTGCCGCTGGAATAGGAACAGGGACTACTGCTCTAATGGCCACT

GlnGlnPheGlnGlnLeuGlnAlaAlaValGlnAspAspLeuArgGluValGluLysSerIleSerAsnLeuGluLysSerLeuThrSerLeuSerGluValVal
CAGCAATTCCAGCAGCTCCAAGCCGCAGTACAGGATGATCTCAGGGAGGTTGAAAAATCAATCTCTAACCTAGAAAAGTCTCTCACTTCCCTGTCTGAAGTTGTC
                                                                                  G

LeuGlnAsnArgArgGlyLeuAspLeuLeuPheLeuLysGluGlyGlyLeuCysAlaAlaLeuLysGluGluCysCysPheTyrAlaAspHisThrGlyLeuVal
CTACAGAATCGAAGGGGCCTAGACTTGTTATTTCTAAAAGAAGGAGGGCTGTGTGCTGCTCTAAAAGAAGAATGTTGCTTCTATGCGGACCACACAGGACTAGTG
                                             ⌐——18——⌐

ArgAspSerMetAlaLysLeuArgGluArgLeuAsnGlnArgGlnLysLeuPheGluSerThrGlnGlyTrpPheGluGlyLeuPheAsnArgSerProTrpPhe
AGAGACAGCATGGCCAAATTGAGAGAGAGGCTTAATCAGAGACAGAAACTGTTTGAGTCAACTCAAGGATGGTTTGAGGGACTGTTTAACAGATCCCCTTGGTTT
          C                ⌐—— 25 ——⌐

ThrThrLeuIleSerThrIleMetGlyProLeuIleValLeuLeuMetIleLeuLeuPheGlyProCysIleLeuAsnArgLeuValGlnPheValLysAspArg
ACCACCTTGATATCTACCATTATGGGACCCCTCATTGTACTCCTAATGATTTTGCTCTTCGGACCCTGCATTCTAAATCGATTAGTTCAATTTGTTAAAGACAGG
        ⌐—— 42——⌐                        ⌐—— 57——⌐

IleSerValValGlnAlaLeuValLeuThrGlnGlnPheHisGlnLeuLysProIleGluCysGluProStop          + origin
ATATCAGTGGTCCAGGCTCTAGTTTTGACTCAACAATTTCACCAGCTGAAGCCTATAGAGTGCGAGCCATAGATAAAATAAAAGATTTTATTTAGTTTCCAGAAA
                       ⌐           A          ⌐                        ⌐—— 63 ——⌐
           IR_L        ⌐——— 98B ———⌐
AAGGGGGGAATGAAAGACCCCACCTGTAGGTTTGGCAAGCTAGCTTAAGTAACGCCATTTTGCAAGGCATGGAAAAATACATAACTGAGAATAGAAAAGTTCAGA

TCAAGGTCAGGAACAGATGGAACAGCTGAATATGGGCCAAACAGGATATCTGTGGTAAGCAGTTCCTGCCCCGGCTCAGGGCCAAGAACAGATGGTCCCCAGATG

                                                                                                      DR3
CGGTCCAGCCCTCAGCAGTTTCTAGAAGAACCATCAGATGGTTCCAGGGTGCCCCAAGGACCTGAAATGACCCTGTGCCTTATTTGAACTAACCAATCAGTTCGC
                        ←——————————→                                         ⌐——————— 99 ———————⌐

    DR2        DR1               P
TTCTCGCTTCTGTTCGCGCGCTTCTGCTCCCCGAGCTCAAAAAAAGAGCCCACAACCCCTCACTCGGGGCGCCAGTCCTCCGATTGACTGAGTCGCCCGGGTACC
                                      T              ⌐———— 34————⌐

    poly A
CGTGTATCCAATAAACCCTCTTGCAGTTGCATCCGACTTGTGGTCTCGCTGTTCCTTGGGAGGGTCTCCTCTGAGTGATTGACTACCCGTCAGCGGGGGTCTTTCA
                                                                                              IR_R

TTTGGGGGCTCGTCCGGGATC-3'
  ACCCCCGAGCAGGCCCUA $_A{}^m$
 tRNA$^{pro}$          $^A$C$_{\psi}{}_{\psi}$

FIG. I

FIG. 2

FIG. 3

A  B  C  D

←Pr80*env*

←R

FIG. 4

# FIG. 5

Fragment

Mc-MuLV

AKV

*ArgLeuAsnGlnArgGln*
GCTTAATCAGAGACAG

25

GCTAAAACAACAACAG
*LeuLysGlnGlnGln*

*LeuIleSerThrIleMet*
GATATCTACCATTATG

42

GATATCCACCATCATG
*IleSerThrIleMet*

*LeuMetIleLeuLeuPhe*
AATGATTTTGCTCTTCG

57

GTTAATTTTACTCTTTG
*LeuIleLeuLeuPhe*

*LeuThrGlnGlnPheHisGlnLeuLys*
GACTCAACAATTTCACCAGCTGAAG

98B

GACTCAACAATATCATCAACTTAXG
*ThrGlnGlnTyrHisGlnLeu*

FIG.6

# FIG. 7

| FRAGMENT | POSITION | SEQUENCE |
|---|---|---|
| 1 | 48-81 | CYS LEU GLY GLN ASN SER GLN SER PRO THR SER ASN HIS SER PRO THR SER<br>CYS PRO PRO THR CYS PRO GLY TYR ARG TRP MET CYS LEU ARG ARG PHE ILE |
| 2 | 140-148 | THR LYS PRO SER ASP GLY ASN CYS THR TYR |
| 3 | 2-16 | GLU ASN ILE THR SER GLY PHE LEU GLY PRO LEU LEU VAL LEU GLN CYS |
| 3A | 12-16 | LEU LEU VAL LEU GLN CYS |
| 4 | 22-35 | LEU THR ARG ILE LEU THR ILE PRO GLN SER LEU ASP SER TRP CYS |
| 4A | 31-35 | SER LEU ASP SER TRP CYS |
| 5 | 38-52 | SER LEU ASN PHE LEU GLY GLY THR THR VAL CYS LEU GLY GLN ASN |
| 5A | 47-52 | VAL CYS LEU GLY GLN ASN |
| 6 | 95-109 | LEU VAL LEU LEU ASP TYR GLN GLY MET LEU PRO VAL CYS PRO LEU |
| 6A | 104-109 | LEU PRO VAL CYS PRO LEU |
| 7 | 149-163 | CYS ILE PRO ILE PRO SER SER TRP ALA PHE GLY LYS PHE LEU TRP |
| 8 | 212-226 | PHE LEU PRO LEU LEU PRO ILE PHE PHE CYS LEU TRP VAL TYR ILE |
| 8A | 221-226 | CYS LEU TRP VAL TYR ILE |

EP 0 044 710 B2

## FIG. 8

```
          20              40              60              80
MENITSGFLGPLLVLQAGFFLLTRILTIPQSLDSWWTSLNFLGGTTVCLGQNSQSPTSNHSPTSCPPTCPGYRWMCLRRF

          100             120             140             160
IIFLFILLLCLIFLLVLLDYQGMLPVCPLFPGSSTTSTGPCRTCMTTAQGTSMYPSCCCTKPSDGNCTCIPIPSSWAFGK

          180             200             220
FLWEWASARFSWLSLLVPFVQWFVGLSPTVWLSVIWMMWYWGPSLYSILSPFLPLLPIFFCLWVYI
```

FIG. 9

9

INFLUENZA STRAIN X-47

```
-16                    1              20                    40                      60
(MKTIIALSYIFCLVFA)  QDLPGNDNNSTATLCLGHHAVPNGTLVKTITNDQIEVTNATELVQSSSTGKICNNPHRIL
                     Y————9————                           Y————22————
                     Y————————10————————                        ——13——
                          Y————————11————————                      ——14——
                                                                   ——12——Y

              80              100                120                    140
DGINCTLIDALLGDPHCDGFQNEKWDLFVERSKAFSNCYPYDVPDYASLRSLVASSGTLEFINEGFNWTGVTQNGGSSAC
—22—            Y————16————            ————————18————————
—13—Y              ————————17————————                      Y——33————
————14————————Y                                            Y——23——
        Y——15——                                                  ≡19
                                                                 ≡20
              160              180                200                    220 21
KRGPDSGFFSRLNWLYKSGSTYPVQNVTMPNNDNSDKLYIWGVHHPSTDKEQTNLYVQASGKVTVSTKRSQQTIIPNVGS
————33————            ————————31————C  Y————26————
—23——                       ——31a——C
————————21————————
————20————
——19—Y
              240              260                280                    300
RPWVRGLSSRISIYWTIVKPGDILVINSNGNLIAPRGYFKMRTGKSSIMRSDAPIGTCSSECITPNGSIPNDKPFQNVNK
—26—C

              320
ITYGACPKYVKQNTLKLATGMRNVPEKQTR
————27————
Y = TYROSINE ADDED FOR LABELLING        C = CYSTINE ADDED FOR COUPLING
```

FIG. 10

# FIG. 11

(CYS LEU GLY GLN ASN SER GLN SER PRO THR SER ASN

HIS SER PRO THR SER CYS PRO PRO THR CYS PRO

GLY TYR ARG TRP MET CYS LEU ARG ARG PHE ILE

GLU ASN ILE THR SER GLY PHE LEU GLY PRO

LEU LEU VAL LEU GLN————LEU THR ARG ILE

LEU THR ILE PRO GLN SER LEU ASP SER TRP

LEU VAL LEU LEU ASP TYR GLN GLY MET LEU

PRO VAL CYS PRO LEU)$_n$

n = 1 to 100 or more